# EUROPEAN PATENT SPECIFICATION

(11) **EP 4 572 800 B1**
(45) Date of publication and mention of the grant of the patent: **08.04.2026**
(21) Application number: 24706239.1
(22) Date of filing: 15.02.2024
(51) Int. Cl.: A61K 47/68, A61K 31/495, A61K 51/04, A61P 35/00, C07D 401/14

(54) **TETRAZINES WITH IMPROVED PROPERTIES**
TETRAZINE MIT VERBESSERTEN EIGENSCHAFTEN
TÉTRAZINES PRÉSENTANT DES PROPRIÉTÉS AMÉLIORÉES

(30) Priority: 15.02.2023 EP 23156915; 22.05.2023 EP 23174627; 13.12.2023 EP 23216311; 13.12.2023 EP 23216318; 15.12.2023 EP 23217256; 15.12.2023 EP 23217334; 24.01.2024 EP 24153683
(43) Date of publication of application: 25.06.2025
(73) Proprietor: Tagworks Pharmaceuticals B.V., 6525 ED Nijmegen (NL)
(72) Inventor: KLEIJN, Laurens Henri Johan, 6525 ED Nijmegen (NL); ROBILLARD, Marc Stefan, 6525 ED Nijmegen (NL); ROSSIN, Raffaella, 6525 ED Nijmegen (NL); VERSTEEGEN, Ronny Mathieu, 6525 ED Nijmegen (NL)
(74) Representative: V.O.
(86) International application number: PCT/NL2024/050076
(87) International publication number: WO 2024/172652

(56) References cited:
- WO-A1-2012/085789
- WO-A1-2012/153254
- WO-A1-2020/256546
- WO-A1-2023/158305
- WO-A1-2024/191293
- US-A1- 2022 356 169
- RAFFAELLA ROSSIN ET AL: "Triggered Drug Release from an Antibody-Drug Conjugate Using Fast "Click-to-Release" Chemistry in Mice", BIOCONJUGATE CHEMISTRY, vol. 27, no. 7, 15 June 2016 (2016-06-15), US, pages 1697 - 1706, XP055404901, ISSN: 1043-1802, DOI: 10.1021/acs.bioconjchem.6b00231
- GARCÍA-VÁZQUEZ ROCÍO ET AL: "Development of 18F-Labeled Bispyridyl Tetrazines for In Vivo Pretargeted PET Imaging", PHARMACEUTICALS, vol. 15, no. 2, 18 February 2022 (2022-02-18), pages 245, XP093098473, DOI: 10.3390/ph15020245
- ROSSIN RAFFAELLA ET AL: "Chemically triggered drug release from an antibody-drug conjugate leads to potent antitumour activity in mice", NATURE COMMUNICATIONS, vol. 9, no. 1, 4 May 2018 (2018-05-04), XP055928553, DOI: 10.1038/s41467-018-03880-y

## Description

### Technological field

The disclosure relates to tetrazines with improved properties. Compositions and combinations comprising the tetrazines of the disclosure, as well as methods for using and making same are provided as well.

### Background

In the field of bioorthogonal chemistry the ligation between tetrazines and dienophiles, in particular *trans*-cyclooctenes, has been studied in depth. Rossin et al., Angew. Chem. Int. Ed. 2010, volume 49, pages 3375-3378 describes a tetrazine refered to herein as compound **2.1** having a structure as follows:

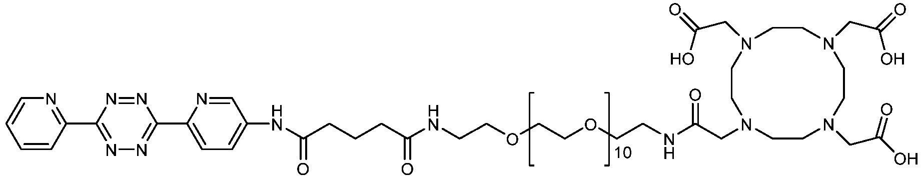

Compound **2.1** and some of its properties were described in further papers on pretargeting, viz. Rossin et al., J. Nucl. Med. 2013, volume 54; pages 1989-1995; Rossin et al., Bioconjugate Chem. 2013, volume 24, pages 1210-1217; Rossin et al., Mol. Pharm. 2014, volume 11, pages 3090-3096; Van Duijnhoven et al., J. Nucl. Med. 2015, volume 56, pages 1422-1428; and Edem et al. Molecules 2020, volume 25, page 463.

While the ligation works well both *in vitro* as *in vivo,* identifying new optimal compounds for clinical use remains a research focus. Along these lines it is desired to identify new tetrazines with overall good *in vitro* and *in vivo* properties, *e.g.* one or more of: good stability, good reactivity with and/or high payload release from trans-cyclooctenes (especially *in vivo*), low membrane permeability, low cell toxicity, and low genotoxicity.

There is thus a need for new tetrazines that address one or more of the abovementioned problems and/or desires.

Further background art includes García-Vázquez Rocío et al., Pharmaceuticals 2022, volume 15, issue 2, page 245; WO 2020/256546; Rossin et al., Bioconjugate Chemistry 2016, volume 27, issue 7, pages 1697-1706; US 2022/356169; WO 2012/085789; and WO 2012/153254.

### Summary

In one aspect, the disclosure relates to a compound or a salt, hydrate, or solvate thereof; wherein said compound has a structure according to Formula (1): 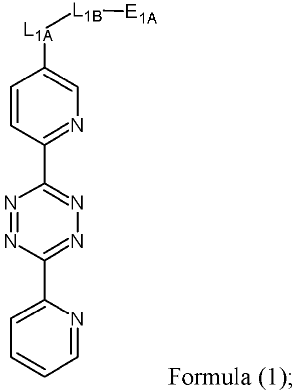 wherein
L_{1A} and L_{1B} are independently selected linkers; E_{1A} is selected from the group consisting of: 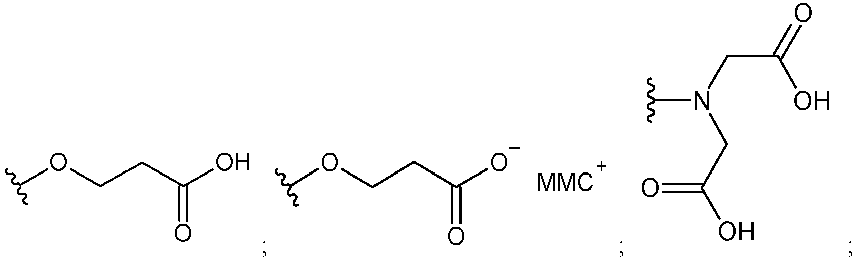 and 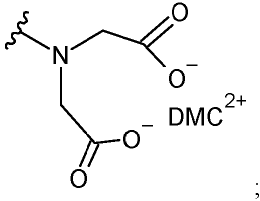 wherein MMC⁺ is a monovalent metal cation; DMC²⁺ is a divalent metal cation; preferably MMC⁺ is Na⁺; preferably DMC²⁺ is Ca²⁺;
with the proviso that the compound of Formula (1) is not 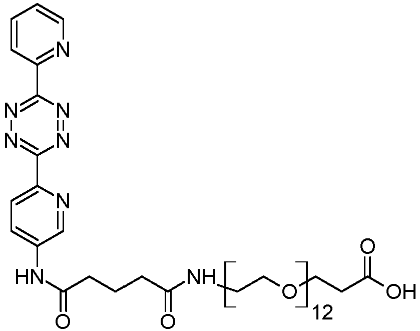 or a salt, solvate, or hydrate thereof

In another aspect, the disclosure pertains to a composition comprising a compound according to Formula (1), or the salt, hydrate, or solvate thereof; preferably the composition is a pharmaceutical composition.

In yet another aspect, the disclosure relates to a combination of (A1) a compound according to Formula (1), or the salt, hydrate, or solvate thereof; and/or (A2) a composition according to the disclosure; with
(B) a dienophile or a salt, solvate, or hydrate thereof; preferably the dienophile comprises a *trans*-cyclooctene moiety.

In a further aspect, the disclosure pertains to the compound according to Formula (1), or the salt, hydrate, or solvate thereof; the composition according to the disclosure; or the combination according to the disclosure; for use as a medicament.

In yet a further aspect, the disclosure relates to the compound according to Formula (1), or the salt, hydrate, or solvate thereof; the composition according to the disclosure; or the combination according to the disclosure; for use in the treatment of a disease in a subject; preferably the subject is a human; preferably the disease is cancer.

In a further aspect still, the disclosure pertains to a non-therapeutic method for reacting:
(ia) the compound according to Formula (1), or the salt, hydrate, or solvate thereof; and/or (iia) the composition according to the disclosure;
with a dienophile or a salt, solvate, or hydrate thereof; wherein said method comprises the step of contacting (ia), and/or (iia) with said dienophile or salt, solvate, or hydrate thereof; preferably said non-therapeutic method is an *in vitro* method; and preferably said dienophile comprises a *trans*-cyclooctene moiety.

In yet a further aspect, the disclosure relates to a non-therapeutic use of:
(a) the compound according to Formula (1), or the salt, hydrate, or solvate thereof;
(b) the composition according to the disclosure and/or
(c) the combination according to the disclosure;
in a click reaction.

In yet a further aspect, the disclosure relates to a method for preparing a compound according to claim 7, wherein said method comprises the steps of:
(a) reacting SM1a or SM1b with a reagent selected from the group consisting of SM2, SM3, and SM4;
(b) if SM1a is used in step (a), subjecting the reaction product of step (a) to oxidation;
(c) optionally, subjecting the reaction product of step (a) or step (b) to salt formation; wherein SM1a, SM1b, SM2, SM3, and SM4 are: 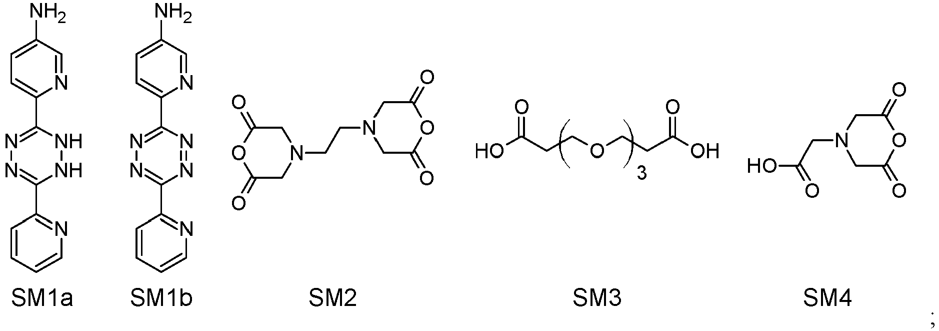 preferably in step (a) SM1a is used; preferably in step (b) the reaction product of step (a) is contacted with sodium nitrite.

### Detailed Description

Above mentioned compound **2.1** was first described by Rossin et al., Angew. Chem. Int. Ed. 2010, volume 49, pages 3375-3378. Therein, said compound was used in relation to pretargeting of tumors, and selected for its good pharmacokinetics, reactivity and sufficient stability *in vivo.*

Furthermore, compound **2.1** has also been used *in vivo* to bind and detect unreacted *trans*-cyclooctenes by Rossin et al., Bioconjugate Chem. 2016, volume 27, pages 1697-1706; and Rossin et al., Nature Commun. 2018, volume 9, article 1484.

Additionally, compound **2.1** has some very interesting properties for *in vivo* release of a payload, typically a drug, attached to a *trans-*cyclooctene*.* Compound **2.1** as a higher reactivity towards *trans*-cyclooctenes than other tetrazines and has a good clearance rate (Rossin et al., Nature Commun. 2018, volume 9, article 1484, page 6, left column). Although compound **2.1** has a low release yield with most *trans*-cyclooctenes, like most bis-(2-pyridyl)-tetrazines, the release yield for bis-(2-pyridyl)-tetrazines is (near-)quantitative when using *trans*-cyclooctenes with certain substituents (WO 2020/256546, in particular Example 5 at pages 294-296). As such, compound **2.1** is also a strong candidate for use in payload-release *in vivo.* Consequently, compound **2.1** is the best-studied tetrazine for *in vivo* use in literature, and has been reported to be a promising candidate for clinical use.

However, properties of compound **2.1** that could be improved upon have been identified by the inventors and reported on herein for the first time. Such properties may arise in certain conditions when using **2.1** *in vivo* as an activator for the payload release from *trans-*cyclooctenes, which typically requires higher doses than when using **2.1** for radioimaging and/or radiotherapy.

First, it was found that compound **2.1** strongly inhibits the physiologically relevant enzymes cyclooxygenase (COX-1), acetyl cholinesterase (ACES), monoamine oxidase (MAO-B), and calcium channel L-type, dihydropyridine. Each of these proteins is important in maintaining health in a subject, and undesired inhibition of these enzymes and/or transporter may lead to side-effects. Based on this, it is desired that tetrazines be provided that show lower inhibition for one or more of these enzymes and/or transporter.

Second, it was found that **2.1** has a maximum tolerated dose (MTD) in mice of about 39 µmol/kg. This may limit the therapeutic window in which **2.1** can be safely used *in vivo.* It is therefore also desired that new tetrazines with a higher maximum tolerated dose be provided.

Furthermore, the synthesis of **2.1** comprises multiple steps, and it is desired that tetrazines be provided that can be synthesized in fewer steps and/or in a more straightforward way.

Some aspects and embodiments of the disclosure are therefore, in a broad sense, based on the judicious insight that tetrazines of Formula (1) may meet one or more of the aforementioned desires.

In particular, the tetrazines of Formula (1) can show lower inhibition of the enzymes cyclooxygenase (COX-1), acetyl cholinesterase (ACES), monoamine oxidase (MAO-B), and/or calcium channel L-type, dihydropyridine, as shown in Example 3 herein.

Moreover, the tetrazines of Formula (1) typically have a relatively higher maximum tolerated dose in mice, as shown in Example 4 herein.

Furthermore, the tetrazines of Formula (1) can be readily synthesized in fewer steps than required for **2.1,** as shown in Example 2 herein.

Finally, the tetrazines of Formula (1) in many embodiments also show further excellent *in vitro* and *in vivo* properties, as shown in Example 5 herein.

Preferred embodiments of Formula (1) are further described below. All of these embodiments can be combined as long as they are not mutually exclusive.

### Compounds of Formula (1)

The compounds of the disclosure are according to Formula (1) as defined above. For ease of reference, compounds of Formula (1) are below described as *e.g*. "compounds of the disclosure". It will be understood that also the salt, hydrate, or solvate of said compounds are included by such a statement.

In relation to Formula (1), MMC⁺ is preferably selected from the group consisting of Na⁺, K⁺, and Cu⁺. Most preferably, MMC⁺ is Na⁺.

In relation to Formula (1), DMC²⁺ is preferably selected from the group consisting of Ca²⁺, Mg²⁺, Mn²⁺, Zn²⁺, Cu²⁺, Cd²⁺, Cr²⁺, Co²⁺, Fe²⁺, Pb²⁺, Ni²⁺, Hg²⁺, Sn²⁺ and Pt²⁺. Most preferably, DMC²⁺ is Ca²⁺.

In Formula (1), L_{1A} and L_{1B} are independently selected linkers.

Preferably, L_{1A} is a linker containing at most 10 atoms. More preferably, L_{1A} is selected from the group consisting of -O-, -S-, -SS-, -NR_{L1A}-, -N=N-, -C(O)-, -C(O)NR_{L1A}-, - OC(O)-, -C(O)O-, -OC(O)NR_{L1A}-, -NR_{L1A}C(O)-, -NR_{L1A}C(O)O-, -NR_{L1A}C(O)NR₄-, -SC(O)-, -C(O)S-, -SC(O)O-, -OC(O)S-, -SC(O)NR_{L1A}-, and -NR_{L1A}C(O)S-. Even more preferably, L_{1A} is -NR_{L1A}C(O)-, and most preferably L_{1A} is -NHC(O)-.

In relation to Formula (1), R_{L1A} is hydrogen or C₁₋₃ alkyl. Preferably R_{L1A} is hydrogen.

Preferably, L_{1B} is a linker containing at most 50 atoms, more preferably L_{1B} is a linker containing at most 30 atoms; even more preferably L_{1B} is a linker containing at most 25 atoms; and most preferably L_{1B} is a linker containing at most 10 atoms.

Preferably, L_{1B} is selected from the group consisting of -CH₂-, 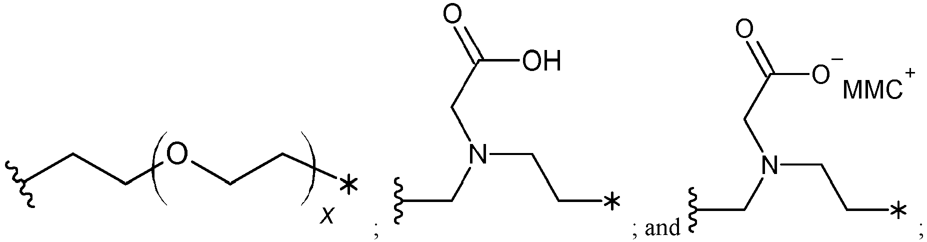 wherein the wiggly line indicates a bond to L_{1A} and the asterisk a bond to E_{1A}.

In relation to L_{1B} x is an integer in a range of from 1 to 12; preferably x is an integer in a range of from 2 to 7; and more preferably x is an integer in a range of from 2 to 4. Most preferably, x is 3.

Preferably, L_{1B} and E_{1A} together form a moiety selected from the group consisting of:

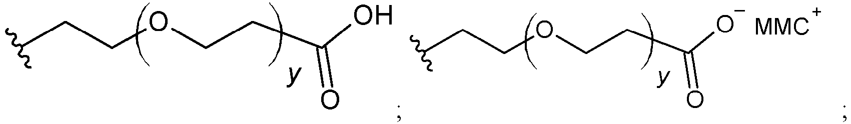

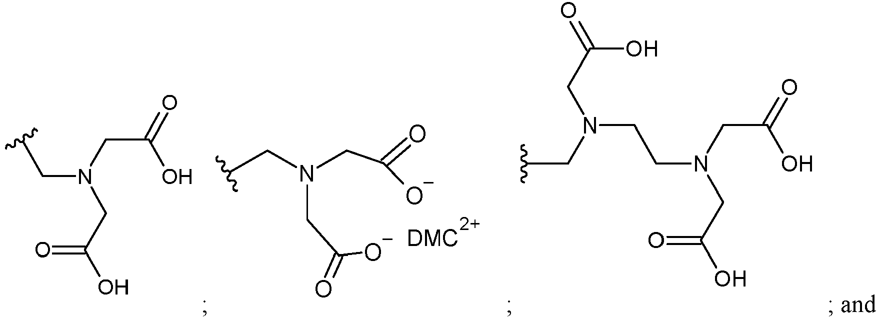

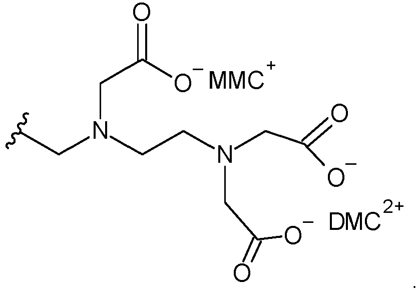

In relation to Formula (1), y is an integer in a range of from 1 to 13; preferably y is an integer in a range of from 1 to 11; more preferably y is an integer in a range of from 2 to 9; more preferably y is an integer in a range of from 2 to 7; more preferably y is an integer in a range of from 2 to 6; more preferably y is an integer in a range of from 2 to 4. Most preferably, y is 3.

Preferably, the compounds of Formula (1) do not comprise ¹⁸F, more preferably the compounds of Formula (1) do not comprise a radionuclide.

Preferably, the compound of Formula (1) is selected from the group consisting of:

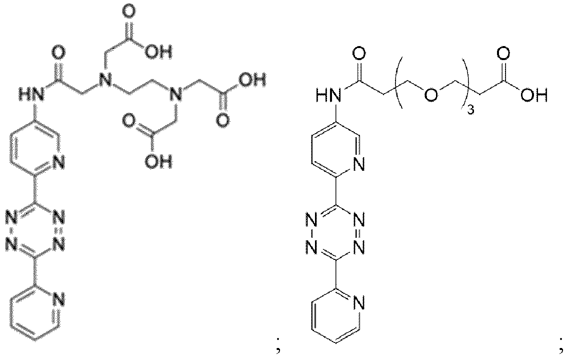

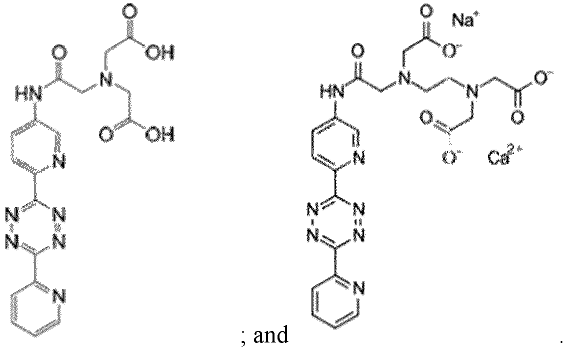

More preferably, the compound of Formula (1) is:

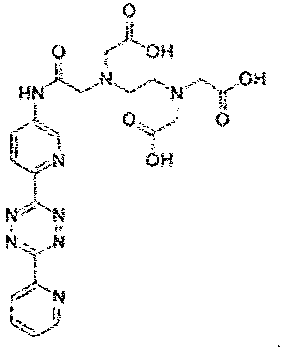

More preferably, the compound of Formula (1) is:

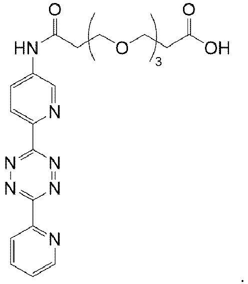

More preferably, the compound of Formula (1) is:

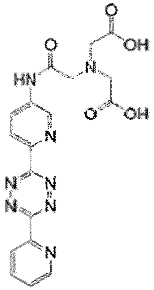

Most preferably, the compound of Formula (1) is:

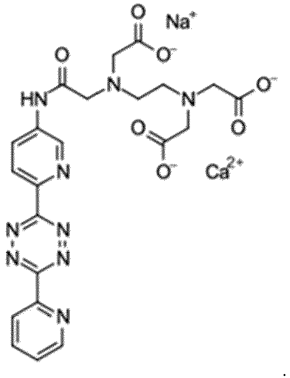

### Compositions of the disclosure

The disclosure also pertains to a composition comprising a compound according to Formula (1), or the salt, hydrate, or solvate thereof. Preferably, the composition is a pharmaceutical composition. Preferably, the composition of the disclosure further comprises a pharmaceutically acceptable carrier. It is also preferred that if a salt of a compound of Formula (1) is included in the composition of the disclosure, a pharmaceutically acceptable salt is used.

### Combinations of the disclosure

The disclosure also relates to a combination of (A1) a compound according to Formula (1), or the salt, hydrate, or solvate thereof; and/or (A2) a composition according to the disclosure; with (B) a dienophile or a salt, solvate, or hydrate thereof.

Preferably, the combination of the disclosure is a kit. More preferably, the combination of the disclosure is a kit wherein (A1) and/or (A2) is/are physically separated from (B).

Preferably, a dienophile as used herein comprises an eight-membered non-aromatic cyclic mono-alkenylene moiety comprising at least one allylic carbon, and optionally comprising one or more heteroatoms, preferably the heteroatom is N, O, or Si. The eight-membered non-aromatic cyclic mono-alkenylene moiety is optionally substituted. Preferably, the eight-membered non-aromatic cyclic mono-alkenylene moiety is a cyclooctene moiety, more preferably a *trans*-cyclooctene (TCO) moiety. Most preferably, the *trans*-cyclooctene moiety is an all-carbon ring.

Preferably, at least five, more preferably at least six, and most preferably at least seven, members of the eight-membered non-aromatic cyclic mono-alkenylene moiety are not substituted. Preferably, the vinylic carbons are not substituted, *i.e.* are CH. As such, if the dienophile comprises a *trans*-cyclooctene moiety that is an all-carbon ring, then the *trans-*cyclooctene moiety preferably is a ring with one double bond between two CH moieties, *i.e. -* CH=CH-, and the ring further comprises at least three, more preferably at least four, and most preferably at least five CH₂ moieties. The substituted members are preferably N or C, more preferably C.

Preferably, the dienophile comprises a payload that is released upon reacting with a tetrazine of Formula (1). The payload is preferably a drug or a chelating moiety comprising a a radionuclide for imaging. If the dienophile comprises a payload, then at least one allylic carbon of the eight-membered non-aromatic cyclic mono-alkenylene moiety is directly linked to a cleavable bond. The cleavable bond comprises at least one S, N, NH, or O, and is selected from the group consisting of carbamate, thiocarbamate, carbonate, thiocarbonate, ether, ester, thioether, and thioester bonds. More preferably, the cleavable bond is selected from the group consisting of carbamate, ether, and ester bonds. Most preferably, the cleavable bond is a carbamate. The at least one S, N, NH, or O of the cleavable bond is part of the payload, or part of an optional spacer between the cleavable bond and the payload. Preferably, the optional spacer is a self-immolative linker. Self-immolative linkers are well-known in the art.

Preferably, the dienophile in relation to the disclosure is as described in WO 2020/256546, in particular in accordance with any one of claims 1 to 4 thereof, or any embodiment thereof, more preferably as described at page 44, line 5, to page 74, last line, or any embodiment thereof.

Without wishing to be bound by theory, it is believed that optionally present other substituents on the eight-membered non-aromatic cyclic mono-alkenylene moiety do not qualitatively influence the release of the payload upon reaction with a diene. In other words, the payload will be released upon reaction with a diene, regardless of whether other substituents are present on the eight-membered non-aromatic cyclic mono-alkenylene moiety. Several mechanisms for the release of the payload are known in the art. These are for example described in WO 2020/256546, in particular in Scheme 2 on page 39.

Dienophiles can be synthesized by the skilled person on the basis of known synthesis routes to cyclooctenes and corresponding hetero atom(s)-containing rings. The skilled person further is aware of the wealth of cyclooctene derivatives that can be synthesized via the ring closing metathesis reaction using Grubbs catalysts. As mentioned above, the TCO possibly includes one or more heteroatoms in the ring. This is as such sufficiently accessible to the skilled person [e.g. WO2016025480]. Reference is made, e.g., to the presence of a thioether in TCO: [Cere et al. J. Org. Chem. 1980, 45, 261]. Also, e.g., an - O-SiR₂-O moiety in TCO: [Prevost et al. J. Am. Chem. Soc. 2009, 131, 14182]. References to TCO syntheses wherein the allylic positioned leaving group (R₄₈) is an ether, ester, carbonate, carbamate or a thiocarbamate are: [Versteegen et al Angew. Chem. Int. Ed. 2018, 57, 10494], and [Steiger et al Chem Comm 2017, 53, 1378].

Preferably, the dienophile is a compound or a salt, hydrate, or solvate thereof; wherein said compound has a structure according to Formula (2): 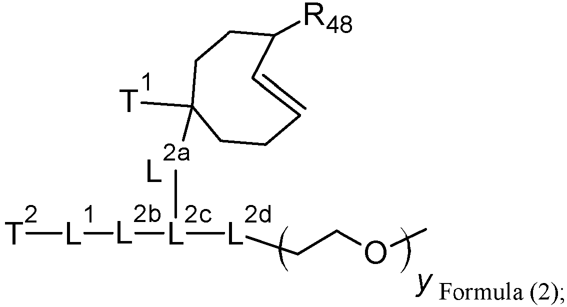 wherein
L¹ is selected from the group consisting of linear or branched C₄-C₁₂ alkylene, C₃-C₈ (hetero)cycloalkylene, C₆-C₁₂ arylene, and C₄-C₁₁ heteroarylene; preferably L¹ is linear or branched C₄-C₁₂ alkylene, more preferably L¹ is linear or branched C₄-C₁₀ alkylene, and most preferably L¹ is linear C₅-C₆ alkylene;
L^{2a}, L^{2b}, and L^{2d} are each independently selected from the group consisting of - C(O)NL^{2T}-, -NL^{2T}C(O)-, -O-, -S-, -NL^{2T}-, -N=N-, and -C(O)-; wherein L^{2T} is hydrogen or methyl, preferably L^{2T} is hydrogen;
L^{2c} is selected from the group consisting of C₁-C₈ (hetero)alkanetriyl, C₅-C₆ (hetero)arenetriyl, C₃-C₇ cycloalkanetriyl, and C₂-C₇ heterocycloalkanetriyl; preferably L^{2c} is C₁-C₈ (hetero)alkanetriyl, more preferably L^{2c} is C₁-C₈ alkanetriyl, and most preferably L^{2c} is C₄-C₆ alkanetriyl;
T¹ is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃; preferably T¹ is -OT^{1A}; and most preferably T¹ is -OH; each T^{1A} is independently selected from the group consisting of hydrogen, (hetero)alkyl, (hetero)alkenyl, (hetero)alkynyl, (hetero)aryl, and an amino acid residue; preferably T^{1A} is hydrogen or methyl, more preferably T^{1A} is hydrogen;
T² is a bioconjugation moiety or a group -L³-C^{B}; preferably the bioconjugation moiety is N-maleimidyl; L³ is a residue of a bioconjugation moiety; preferably L³ is a residue of a maleimidyl moiety or a residue of an N-hydroxysuccinimidyl moiety;
C^{B} is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof; preferably C^{B} is a protein, more preferably C^{B} is an antibody or a diabody, even more preferably C^{B} is a diabody, and most preferably C^{B} is AVP0458 consisting of two monomers, wherein each of the two monomers has an amino acid sequence according to SEQ ID NO: 1;
y is an integer in a range of from 1 to 50; preferably y is an integer in a range of from 10 to 40, more preferably in a range of from 12 to 37, even more preferably in a range of from 15 to 35, more preferably still in a range of from 20 to 30, and most preferably in a range of from 23 to 25; and
R₄₈ is a releasable group, preferably R⁴⁸ is -O-CO-C^{A}; wherein C^{A} is a payload, preferably C^{A} is a drug; preferably the drug is linked to the moiety -O-CO- via a secondary or tertiary nitrogen atom that is part of the drug, forming a carbamate; preferably the drug is monomethyl auristatin E (MMAE).

More preferably, the compound according to Formula (2) is a compound is according to Formula (3): 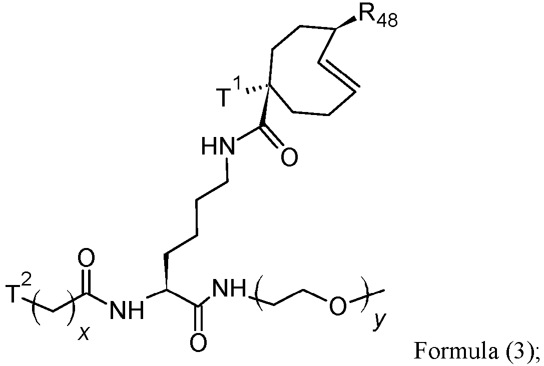 wherein x is an integer in a range of from 4 to 12; preferably x is an integer in a range of from 4 to 8, more preferably x is an integer in a range of from 4 to 6.

In Formula (2) and Formula (3), it is preferred that T² is selected from the group consisting of 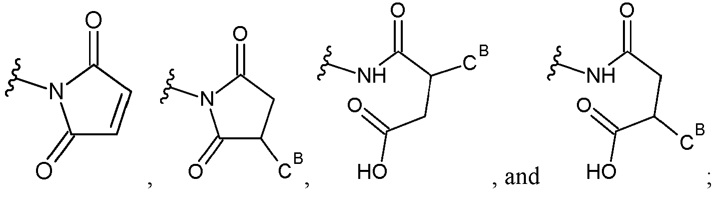 wherein
C^{B} is a protein.

Even more preferably, the dienophile is: 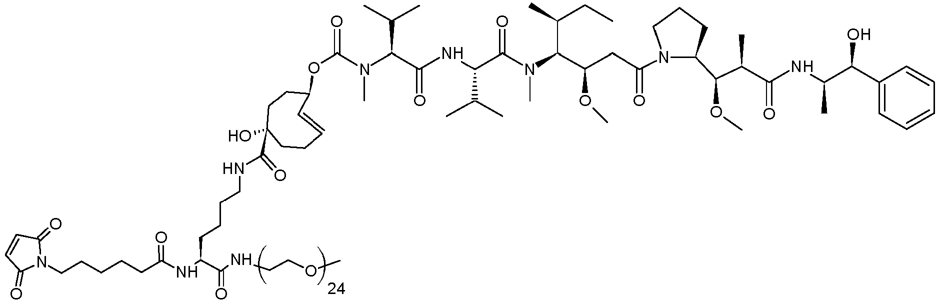 ; or 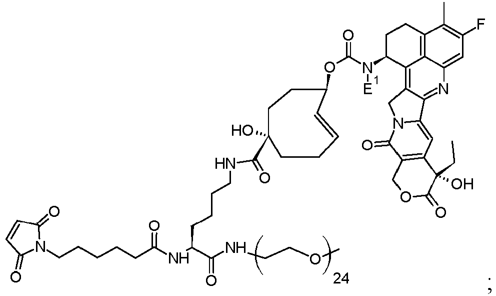 or a salt, hydrate, or solvate thereof.

As used herein in relation to dienophiles of the disclosure **E¹** is -H or -CH₃.

More preferably still, the dienophile is: 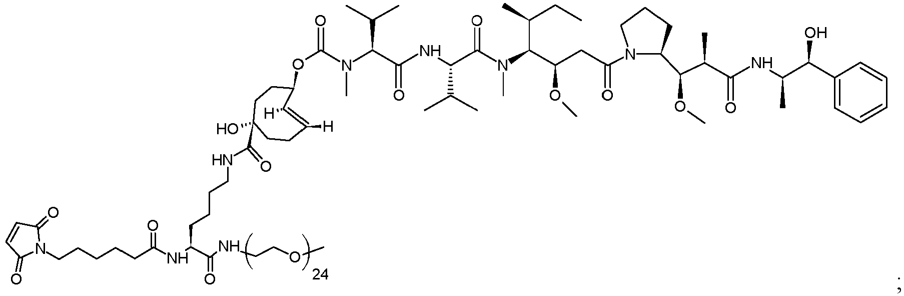 or 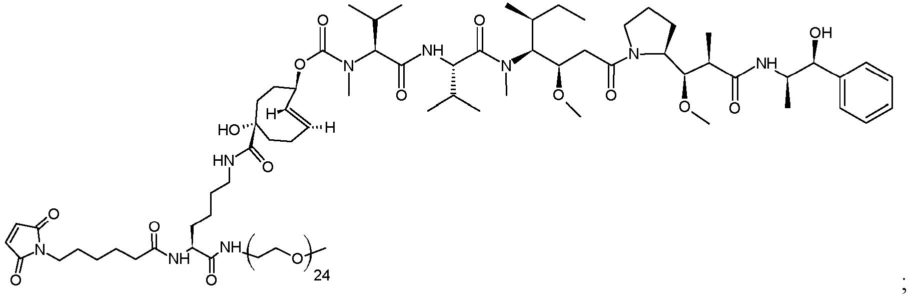 or 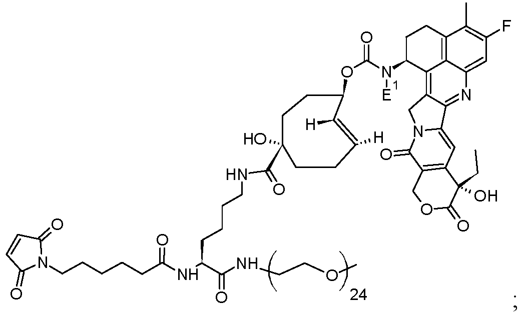 or 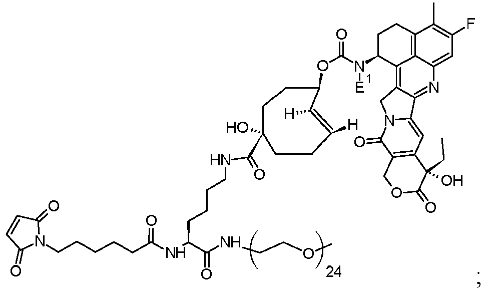 or a salt, hydrate, or solvate thereof.

Yet more preferably, the dienophile is: 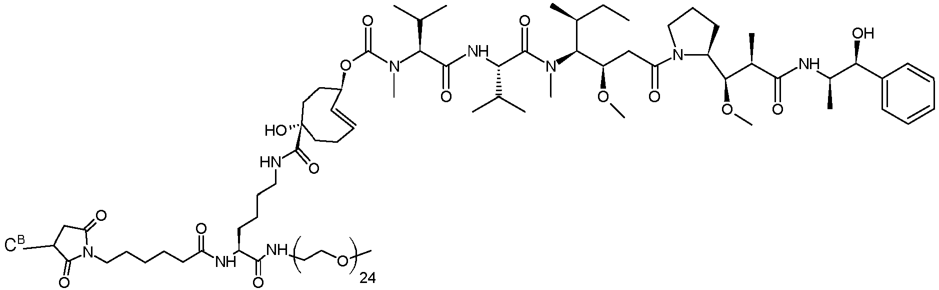 ; or 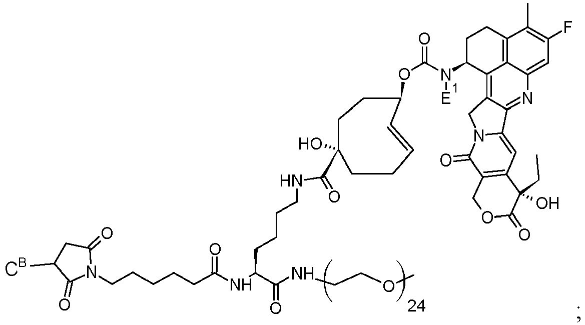 wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine.

Most preferably, the dienophile is: 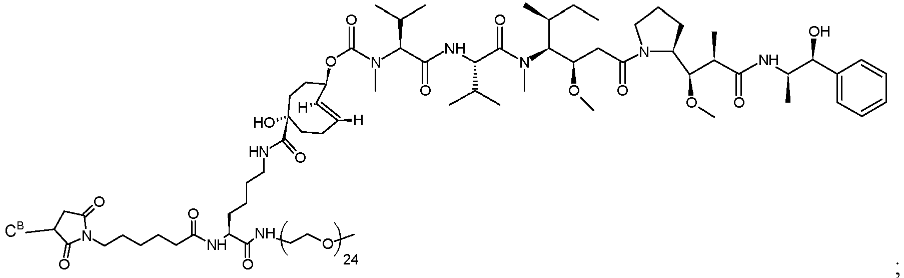 or 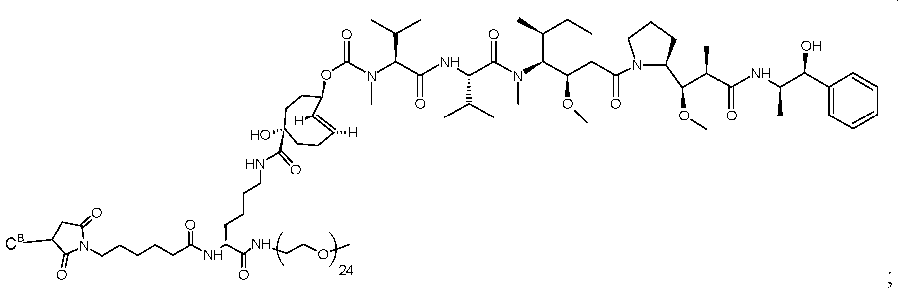 or 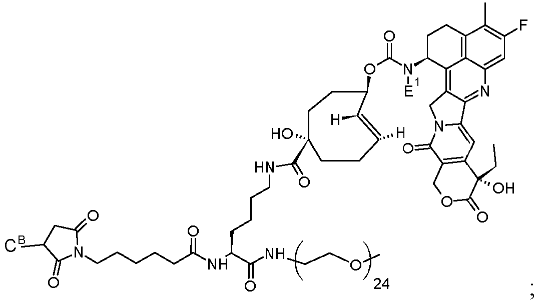 or 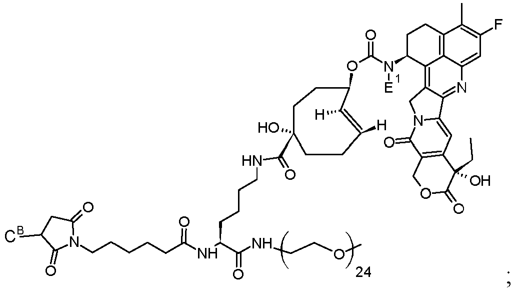 or a salt, hydrate, or solvate therof; wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine.

In other preferred embodiments, the dienophile is a conjugate, or a salt, hydrate, or solvate thereof. Preferably, the conjugate is: 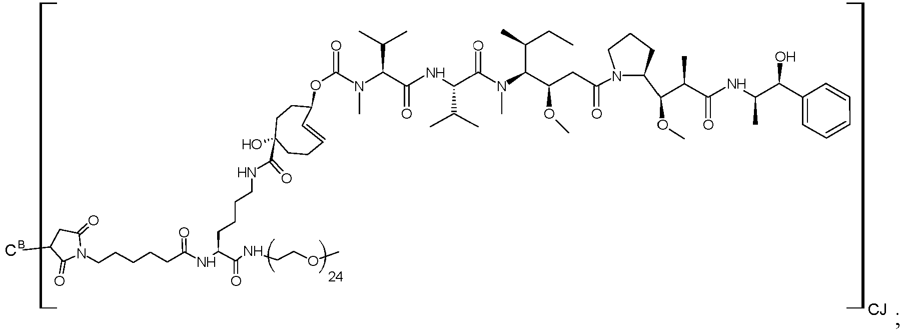 or 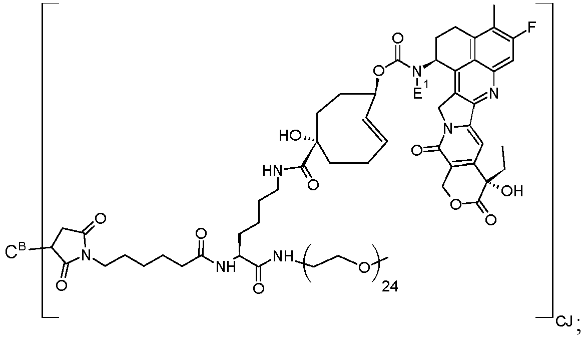 wherein CJ is in a range of from 1 to 12; wherein C^{B} is AVP0458 consisting of two monomers, wherein each of the two monomers has an amino acid sequence according to SEQ ID NO: 1; preferably CJ is of from 2 to 10, more preferably of from 2.5 to 8, even more preferably of from 3 to 6, even more preferably still of from 3.5 to 4, and most preferably about 4; preferably C^{B} is linked to each maleimidyl group via a sulfur atom, preferably the sulfur atom is part of a cysteine.

More preferably, the conjugate is: 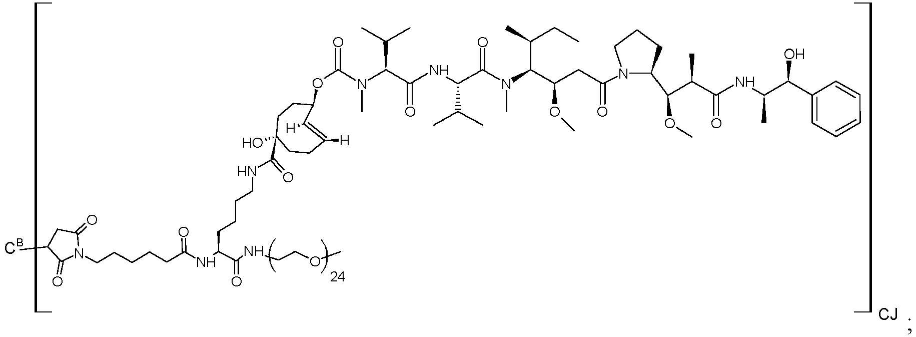 or 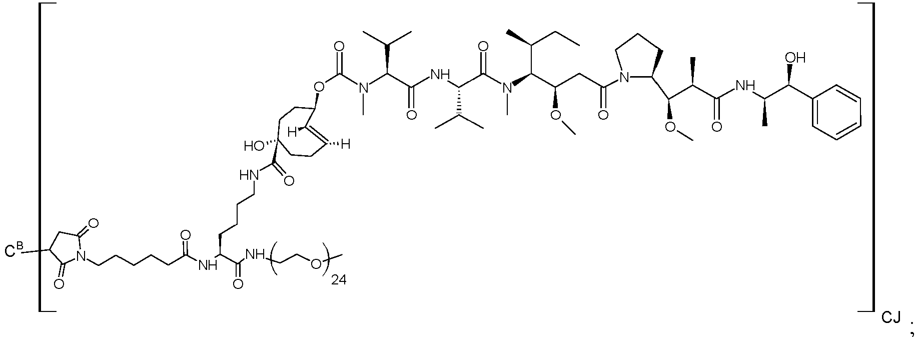 or 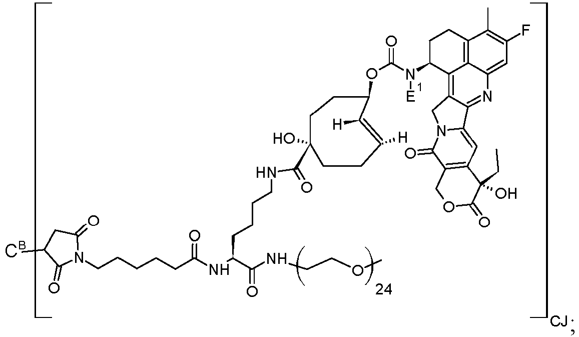 or 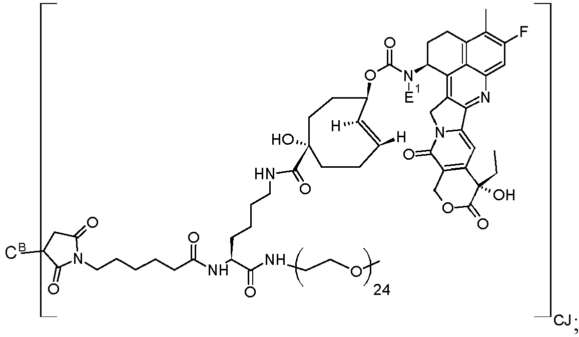 or a salt, hydrate, or solvate thereof; wherein CJ is in a range of from 1 to 12; wherein C^{B} is AVP0458 consisting of two monomers, wherein each of the two monomers has an amino acid sequence according to SEQ ID NO: 1; preferably CJ is of from 2 to 10, more preferably of from 2.5 to 8, even more preferably of from 3 to 6, even more preferably still of from 3.5 to 4, and most preferably about 4; preferably C^{B} is linked to each maleimidyl group via a sulfur atom, preferably the sulfur atom is part of a cysteine.

### AVP0458

As used herein, AVP0458 refers to a TAG72-binding diabody derived from the CC49 antibody. AVP0458 is a diabody consisting of two monomers, each monomer having an amino acid sequence according to SEQ ID NO: 1:

SEQ ID NO:1 (amino acid sequence of AVP0458 diabody monomer):
SVQLQQSDAELVKPGASVKISCKASGYTFTDHAIHWVKQNPEQGLEWIGYFSPGNDD
FKYNERFKGKATLTADKSSSTAYLQLNSLTSEDSAVYFCTRSLNMAYWGQGTSVTV
SSGGGGSDIVMTQSCSSCPVSVGEKVTLSCKSSQSLLYSGNQKNYLAWYQQKPGQSP
KLLIYWASTRESGVPDRFTGSGSGTDFTLSISSVETEDLAVYYCQQYYSYPLTFGAGT
KLVLKR

Herein, the underlining indicates the cysteines that are preferably modified with or linked to a dienophile of the disclosure or the remainder thereof if AVP0458 is itself part of the dienophile of the disclosure.

Thus, in SEQ ID NO: 1 it is preferred that at least one of the underligned cysteines, more preferably both underlined cysteines, is modified with or linked to a dienophile according to the disclosure. In other words: it is preferred that the sulfur atom of the underlined cysteines is coupled to a moiety T² as defined herein, preferably T² is the residue of an N-maleimidyl group.

### Non-therapeutic methods using and uses for using compounds of Formula (1)

In some embodiments, the disclosure pertains to non-therapeutic methods and non-therapeutic uses. Preferably, the dienophile used therein is as described in relation to the combination of the disclosure.

For the non-therapeutic method of the disclosure it is preferred that the compound of Formula (1) (viz. (ia)), and/or the composition of the disclosure (viz. (iia)), and the dienophile are further contacted with a solvent. The skilled person is aware of suitable solvents for a reaction between a tetrazine and a dienophile. Preferably, the solvent comprises water, and more preferably the solvent is water.

For the non-therapeutic use, the click reaction is preferably a bioorthogonal click reaction. Preferably, the click reaction is performed *in vitro,* although non-therapeutic reactions *in vivo* can be carried out as well.

### Methods for preparing compounds of Formula (1)

The disclosure also relates to a method for preparing preferred compounds of Formula (1). In step (a) thereof, SM1a or SM1b is reacted with a reagent selected from the group consisting of SM2, SM3, and SM4. Preferably, SM1a is used in step (a).

If SM1a is used in step (a), then step (b) is carried out after step (a). In step (b), the reaction product of step (a) is subjected to oxidation. Preferably, in step (b) the oxidation is performed by adding sodium nitrate, optionally in the presence of an acid, preferably formic acid. If SM1b is used in step (a), then step (b) does not need to be carried out.

In the entirely optional step (c) the reaction product of step (a) or step (b) is subjected to salt formation. The skilled person is aware of standard procedures to form salts from compounds comprising one or more carboxylic acid groups. Preferably, sodium and/or calcium salts are formed in step (c).

For all of steps (a), (b), and (c), it is preferred that the reagents are contacted with a solvent. The skilled person is aware of suitable solvents to be used in said steps.

The present disclosure is herein described with respect to particular embodiments, but the disclosure is not limited thereto but only by the claims. Where an indefinite or definite article is used when referring to a singular noun e.g. "a" or "an", "the", this includes a plural of that noun unless something else is specifically stated.

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

Thus, the scope of the expression "a device comprising means A and B" should not be limited to devices consisting only of components A and B. It means that with respect to the present disclosure, the only relevant components of the device are A and B.

The compounds disclosed herein may occur in different tautomeric forms. The compounds disclosed herein are meant to include all tautomeric forms, unless stated otherwise. When the structure of a compound is depicted as a specific tautomer, it is to be understood that the disclosure of the present application is not limited to that specific tautomer, unless stated otherwise.

Unless stated otherwise, the compounds disclosed herein and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

In several formulae, groups or substituents are indicated with reference to letters such as "A", "B", "X", "Y", and various (numbered) "R" groups. In addition, the number of repeating units may be referred to with a letter, e.g. n in -(CH₂)ₙ-. The definitions of these letters are to be read with reference to each formula, i.e. in different formulae these letters, each independently, can have different meanings unless indicated otherwise.

Herein, reference is made to "alkyl", and the like. The number of carbon atoms that these groups have, excluding the carbon atoms comprised in any optional substituents as defined below, can be indicated by a designation preceding such terms (e.g. "C₁-C₈ alkyl" means that said alkyl may have from 1 to 8 carbon atoms). For the avoidance of doubt, a butyl group substituted with a -OCH₃ group is designated as a C₄ alkyl, because the carbon atom in the substituent is not included in the carbon count.

As used herein, alkyl groups are unsubstituted and have the general formula CₙH₂ₙ₊₁ and may be linear or branched. Examples of alkyl groups include methyl, ethyl, propyl, 2-propyl, t-butyl, 1-hexyl, 1-dodecyl, etc. Thus, C₁₋₃ alkyl groups are methyl, ethyl, 1-propyl, and 2-propyl.

The term "salt thereof" means a compound formed when an acidic proton, typically a proton of an acid, is replaced by a cation, such as a metal cation or an organic cation and the like. The term "salt thereof" also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted salt" means a salt that is acceptable for administration to a patient, such as a mammal (salts with counter-ions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

It will be understood that herein, the terms "moiety" and "group" are used interchangeably when referring to a part of a molecule.

It will be understood that when a heteroatom is denoted as -X(R')₂-, wherein X is the heteroatom and R' is a certain moiety, then this denotes that two moieties R' are attached to the heteroatom.

It will be understood that when a group is denoted as, for example, -((R₅₁)₂-R₅₂)₂- or a similar notation, in which R₅₁ and R₅₂ are certain moieties, then this denotes that first, it should be written as -R₅₁-R₅₁-R₅₂-R₅₁-R₅₁-R₅₂- before the individual R₅₁ and R₅₂ moieties are selected, rather than first selecting moieties R₅₁ and R₅₂ and then writing out the formula.

### Examples

### Example 1: General methods

All reagents, chemicals, materials and solvents were obtained from commercial sources and were used as received, including nitrile starting compounds that have not been described. All solvents were of AR quality. Analytical thin layer chromatography (TLC) was performed on Kieselgel F-254 precoated silica plates. Column chromatography was carried out on Screening Devices B.V. silica gel (flash: 40-63 µm mesh; normal: 60-200 µm mesh). Reverse phase chromatography was performed using a Büchi Reveleris C18 column (80 g). ¹H NMR and ¹³C NMR spectra were recorded on a Bruker Avance III HD (400 MHz for ¹H NMR and 100 MHz for ¹³C NMR) spectrometer or a JEOL (500 MHz for ¹H NMR) at 298 K. Chemical shifts are reported in ppm downfield from TMS at rt. Abbreviations used for splitting patterns are s = singlet, d = doublet, dd = double doublet, t = triplet, q = quartet, m = multiplet and br = broad. HPLC-PDA/MS was performed using a Shimadzu LC-10 AD VP series HPLC coupled to a diode array detector (Finnigan Surveyor PDA Plus detector, Thermo Electron Corporation) and an Ion-Trap (LCQ Fleet, Thermo Scientific). HPLC-analyses were performed using a Alltech Alltima HP C₁₈ 3µ column using an injection volume of 1-4 µL, a flow rate of 0.2 mL/min and typically a gradient (5 % to 100 % in 10 min, held at 100 % for a further 3 min) of acetonitrile (MeCN) in H₂O (both containing 0.1 % formic acid) at 298 K.

### Example 2: Synthesis of tetrazines

### Example 2.1: Reference compound 2.1

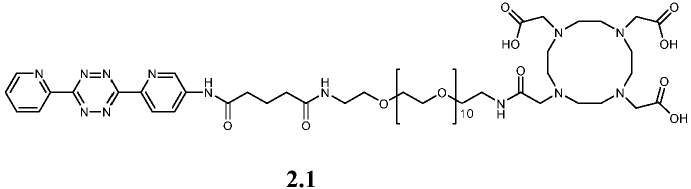

The synthesis of 2,2',2''-(10-(2,40,44-Trioxo-44-((6-(6-(pyridine-2-yl)-1,2,4,5-tetrazin-3-yl)pyridine-3-yl)amino)-6,9,12,15,18,21,24,27,30,33,36-undecaoxa-3,39-diazatetratetracontyl)-1,4,7,10-tetraazacyclododecane-1,4,7-triyl)triacetic acid **(2.1)** was reported in Rossin et al., Angew. Chem. Int. Ed. 2010, 49, 3375 -3378.

### Example 2.2: 2,2'-((2-((Carboxymethyl)(2-oxo-2-((6-(6-(pyridin-2-yl)-1,2-dihydro-1,2,4,5-tetrazin-3-yl)pyridin-3-yl)amino)ethyl)amino)ethyl)azanediyl)diacetic acid (2.3)

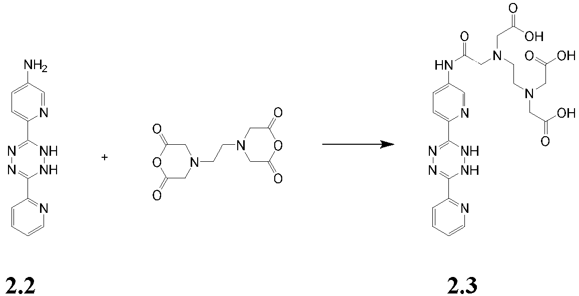

Compound **2.2** was prepared according to Blackman et al. J. Am. Chem. Soc. 2008, 130, 13518-13519. Ethylenediaminetetraacetic dianhydride (1.86 g, 7.26 mmol) was dissolved in dry DMSO (3 mL) by gentle heating. The mixture was allowed to cool to room temperature and a solution of **2.2** (450 mg, 1.78 mmol) in DMSO (11 mL) was slowly added. The orange, hazy mixture was stirred at room temperature under an atmosphere of argon for 5 h. Subsequently water (0.1 mL) was added and the mixture was stirred for 30 min. **2.3** mixture was used without further purification. ESI-MS: m/z Calc. for C₂₂H₂₅N₉O₇ 527.19 Da; Obs. [M+H]⁺ 528.42 Da and [M-H⁺]⁻ 526.50 Da.

### Example 2.3: 2,2'-((2-((Carboxymethyl)(2-oxo-2-((6-(6-(pyridin-2-yl)-1,2,4,5-tetrazin-3-yl)pyridin-3-yl)amino)ethyl)amino)ethyl)azanediyl)diacetic acid (2.4)

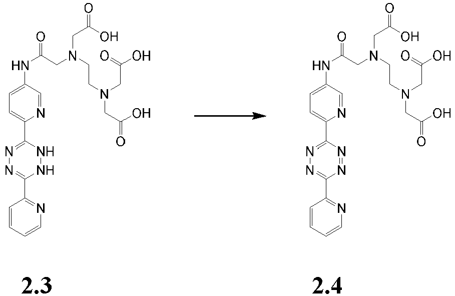

The crude reaction mixture of **2.3** was diluted with water (15 mL) and acidified by addition of formic acid (0.2 mL). Sodium nitrite (400 mg, 5.79 mmol) was added and the pink mixture was stirred in a closed flask at room temperature for 1 h. An aqueous solution of 1 M ammonium acetate (30 mL) was added and the pink suspension was centrifuged at 3000 rpm for 10 min. The clear, dark pink supernatant was isolated and purified by reversed-phase chromatography (C18 column, gradient of 5% MeCN / 0.1 M aqueous ammonium acetate to 25%). The combined product fractions were freeze dried, redissolved in water (25 mL), and again freeze dried and redissolved in water. To the pink solution was added formic acid (0.25 mL), which caused the product to precipitate. The suspension was centrifuged at 3000 rpm for 10 min, after which the clear, faint pink supernatant was discarded. The pink solid was washed with water (25 mL) and centrifuged, for two more times, and then washed with MeCN and centrifuged, for two more times. The remaining pink solid was dried in vacuo to give 486 mg (52% overall yield) of **2.4.** ¹H NMR (400 MHz, DMSO-d₆): δ 12.41 (br.s, 3H), 10.78 (s, 1H), 9.13 (d, J = 2.5 Hz, 1H), 8.94 (dd, J = 4.8, 1.7 Hz, 1H), 8.62 (m, 2H), 8.52 (dd, J = 8.7, 2.5 Hz, 1H), 8.16 (td, J = 7.8, 1.8 Hz, 1H), 7.73 (dd, J = 7.8, 4.7 Hz, 1H), 3.53 (m, J = 10.4 Hz, 8H), 2.85 (s, 4H) ppm. ¹³C NMR (101 MHz, DMSO-d6): δ 173.40, 172.97, 171.94, 163.52, 163.27, 151.08, 150.67, 144.47, 142.13, 138.49, 138.28, 127.05, 126.75, 125.28, 124.66, 58.55, 55.68, 55.37, 52.58, 52.22 ppm. ESI-MS: m/z Calc. for C₂₂H₂₃N₉O₇ 525.17 Da; Obs. [M+H]⁺ 526.33 Da and [M-H]⁻ 524.42 Da.

### Example 2.4: Calcium sodium 2-({2-[bis(carboxylatomethyl)amino]ethyl)[({6-[6-(pyridin-2-yl)-1,2,4,5-tetrazin-3-yl]pyridin-3-yl}carbamoyl)methyl]amino)acetate (2.5)

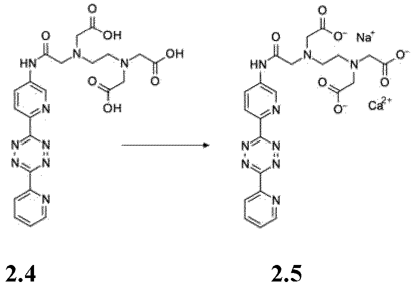

1M Sodium acetate was prepared by dissolving sodium acetate trihydrate in milliQ-H₂O (pH ca. 9.0) followed by acidification to pH=6.4 acidic with AcOH glacial. To an acidic suspension of **2.4** (172 mg, 0.33 mmol; 10 mg/mL) in milliQ-H₂O was added dropwise CaCO₃ (1.67 mL, 0.53 mmol of a 32 mg/mL homogenous suspension). Upon increasing pH, the tetrazine dissolved at pH=5.8 and CaCO₃ addition was halted at pH=6.5. Subsequently, 1.0 M sodium acetate (pH=6.4) was added to the tetrazine to obtain a final 0.1 M sodium acetate concentration. The solution was applied to a Sep-Pak column (10 gr, Waters) for purification (i.e. removal of excess calcium and sodium acetate components). The tetrazine remained at the top of the column and was rinsed once with 0.1 M sodium acetate followed by rinsing with milliQ-H₂O (6 volumes) prior to elution with milliQ-H₂O:MeOH (1:1) aided by vacuum pull. Tetrazine containing fractions were combined, reduced 80% by volume in vacuo, diluted with milliQ-H₂O, and freeze-dried after micropore filtration. The freeze-dried residue was redissolved in milliQ-H₂O at 50 mg/mL, micropore filtered once more, and freeze-dried to obtain **2.5** as a homogenous pink fluffy powder. ¹H NMR (400 MHz, D₂O) δ 8.89 (dd, *J* = 2.6, 0.6 Hz, 1H), 8.79 (ddd, *J* = 4.8*,* 1.7, 0.9 Hz, 1H), 8.63 - 8.55 (m, 2H), 8.37 (dd, *J* = 8.7, 2.6 Hz, 1H), 8.16 (td, *J* = 7.8, 1.7 Hz, 1H), 7.74 (ddd, *J* = 7.7, 4.7, 1.1 Hz, 1H), 3.58 (s, 2H), 3.32 - 3.06 (m, 6H), 2.77 - 2.50 (m, 4H) ppm. ¹³C NMR (100 MHz, D₂O) δ 179.9, 179.3, 174.1, 162.9, 162.5, 150.3, 148.3, 143.9, 142.0, 139.0, 137.3, 128.9, 127.6, 125.3, 124.7, 60.5, 59.8, 54.8 ppm. ESI-MS cal. for C₂₂H₂₃N₉O₇ 525.17 (excl. sodium calcium), found. M+H⁺ 526.25. Elemental anal. calcd for C₂₂H₂₀CaN₉NaO₇: Composition: C (45.1%), Ca (6.8%), N (21.5%), Na (3.9%). Measured: C (44.0%), Ca (7.2%), N (20.5%), Na (3.1%).

### Example 2.5: 3-(2-(2-(3-Oxo-3-((6-(6-(pyridin-2-yl)-1,2-dihydro-1,2,4,5-tetrazin-3-yl)pyridin-3-yl)amino)propoxy)ethoxy)ethoxy)propanoic acid (2.6)

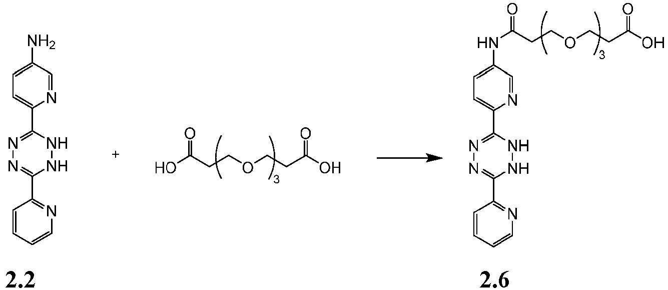

3,3'-((Oxybis(ethane-2,1-diyl))bis(oxy))dipropionic acid (5.90 g, 23.6 mmol) was dissolved in chloroform (100 mL), and pyridinium *p*-toluenesulfonate (0.15 g, 0.597 mmol) and EDC HCl (1.13 g, 5.92 mmol) were added. The solution was stirred at room temperature under an atmosphere of argon for 30 min. Subsequently, compound **2.2** (1.50 g, 5.92 mmol) was added, followed by DMAP (0.36 g, 2.95 mmol). The orange solution was stirred at room temperature under an atmosphere of argon for 90 min, and then washed twice with 0.5 M aqueous citric acid (60 mL). The combined organic layers were dried with sodium sulfate, filtered, and concentrated. Crude **2.6** was used without further purification. ESI-MS: m/z Calc. for C₂₂H₂₇N₇O₆ 485.20 Da; Obs. [M+H]⁺ 486.25 Da and [M-H⁺]⁻ 484.33 Da.

### Example 2.6: 3-(2-(2-(3-Oxo-3-((6-(6-(pyridin-2-yl)-1,2,4,5-tetrazin-3-yl)pyridin-3-yl)amino)propoxy)ethoxy)ethoxy)propanoic acid (2.7)

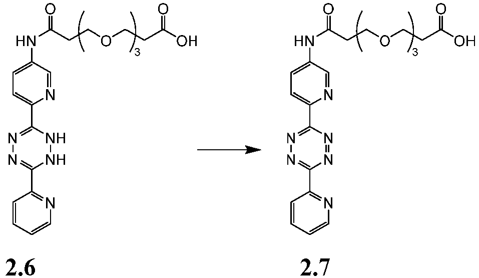

The crude product **2.6** was dissolved in MeCN (30 mL) and water (30 mL), and formic acid (1.5 mL) was added, followed by sodium nitrite (1.23 g, 17.8 mmol). The pink/red solution was stirred in a closed flask at room temperature for 30 min, and then diluted with water (90 mL). The mixture was filtered and purified by reversed-phase chromatography (C18 column, gradient of 15% MeCN / 0.1% aqueous formic acid to 30%). The combined product fractions were freeze-dried to give **2.7** as a pink, fluffy solid (1.32 g, 46% overall yield). ¹H NMR (400 MHz, CDCl₃): δ 9.61 (br.s, 1H), 8.97 (dt, J = 4.6, 1.4 Hz, 1H), 8.79 (m, J = 6.3, 2.6 Hz, 2H), 8.77 - 8.62 (m, 2H), 8.01 (td, J = 7.8, 1.8 Hz, 1H), 7.58 (ddd, J = 7.6, 4.7, 1.2 Hz, 1H), 3.85 (t, J = 5.5 Hz, 2H), 3.80 (t, J = 5.8 Hz, 2H), 3.74 (s, 4H), 3.72 - 3.59 (m, 4H), 2.76 (t, J = 5.5 Hz, 2H), 2.65 (t, J = 5.8 Hz, 2H) ppm. ¹³C NMR (101 MHz, CDCl₃): δ 174.18, 171.32, 163.53, 163.02, 150.91, 150.07, 143.56, 141.93, 138.55, 137.57, 127.36, 126.55, 125.27, 124.38, 70.70, 70.48, 70.20, 67.02, 66.78, 37.91, 35.40 ppm. ESI-MS: m/z Calc. for C₂₂H₂₅N₇O₆ 483.19 Da; Obs. [M+H]⁺ 484.50 Da and [M-H]⁻ 482.33 Da.

### Example 2.7: Sodium 3-(2-{2-[2-({6-[6-(pyridin-2-yl)-1,2,4,5-tetrazin-3-yl]pyridin-3-yl}carbamoyl)ethoxy]ethoxy}ethoxy)propanoate (2.8)

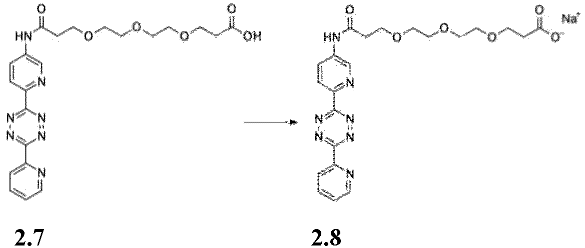

To an acidic suspension of **2.7** (5.65 mg, 10 µmol, 10 mg/mL) in milliQ-H₂O was added dropwise NaHCO₃ (0.13 mL, 13 µmol of a 8.4 mg/mL solution). Upon increasing pH, the tetrazine dissolved at pH=4.5 and NaHCO₃ addition was halted at pH=7.2. The solution was applied to a C18 Sep-Pak column for purification (i.e. removal of excess sodium). The tetrazine remained at the top of the column and was rinsed with milliQ-H₂O (6 volumes) prior to elution with milliQ-H₂O:MeOH (1: 1) aided by vacuum pull. Tetrazine containing fractions were combined, reduced 50% by volume in vacuo and freeze-dried after micropore filter filtration. The freeze-dried residue was redissolved in milliQ-H₂O at 15 mg/mL and freeze-dried to obtain **2.8** as homogenous pink fluffy solid.

### Example 2.8: 2,2'-((2-Oxo-2-((6-(6-(pyridin-2-yl)-1,2-dihydro-1,2,4,5-tetrazin-3-yl)pyridin-3-yl)amino)ethyl)azanediyl)diacetic acid (2.9)

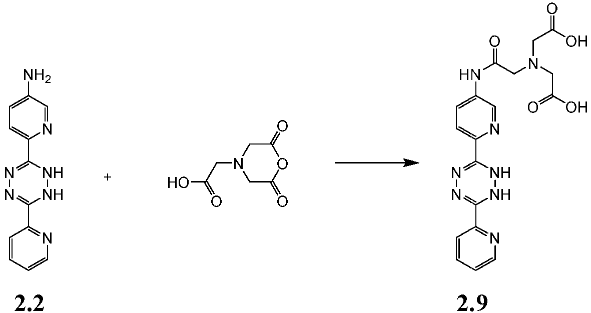

To a solution of **2.2** (168 mg, 0.66 mmol) in DMF (5 mL) was slowly added a solution of nitrilotriacetic anhydride (114 mg, 0.66 mmol) in DMF (1 mL). The solution was stirred at room temperature under an atmosphere of argon for 20 h. Water (30 mL) and formic acid (0.3 mL) were added to precipitate the product, which was isolated by centrifugation and decantation. The precipitate was washed with MeCN (30 mL), and dried in vacuo to yield **2.9** as orange powder (217 mg, 77% yield). ¹H NMR (400 MHz, DMSO-d6): δ 12.63 (br.s, 2H), 10.71 (br.s, 1H), 8.95 (s, 1H), 8.88 (s, 1H), 8.84 (d, J = 2.4 Hz, 1H), 8.64 (dd, J = 4.8, 1.6 Hz, 1H), 8.19 (dd, J = 8.7, 2.5 Hz, 1H), 8.07 - 7.83 (m, 3H), 7.53 (ddd, J = 6.9, 4.8, 1.6 Hz, 1H), 3.58 (s, 4H), 3.55 (s, 2H) ppm. ESI-MS: m/z Calc. for C₁₈H₁₈N₈O₅ 426.14 Da; Obs. [M+H]⁺ 427.33 Da and [M-H]⁻ 425.42 Da.

### Example 2.9: 2,2'-((2-Oxo-2-((6-(6-(pyridin-2-yl)-1,2,4,5-tetrazin-3-yl)pyridin-3-yl)amino)ethyl)azanediyl)diacetic acid (2.10)

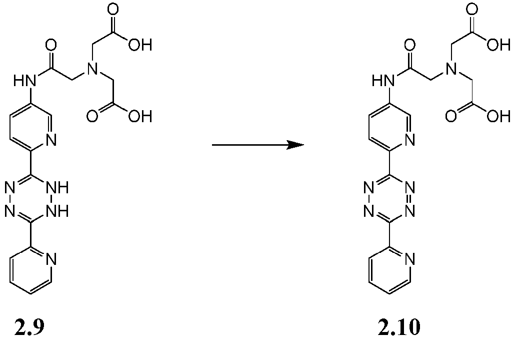

Compound **2.9** (120 mg, 0.282 mmol) was suspended in water (20 mL), and sodium nitrite (97 mg, 1.41 mmol) was added. The suspension was stirred at room temperature under an atmosphere of argon, and turned pink and became clear within 10 min. After 1 h, a precipitate was formed again and the mixture was stored at 4°C for 1 h. The precipitate was isolated by centrifugation, washed with subsequently water (20 mL) and acetonitrile (25 mL), and dried in vacuo, to yield **2.10** as a pink powder (110 mg, 92%). ¹H NMR (400 MHz, DMSO-d6): δ 12.53 (br.s, 2H), 10.92 (br.s, 1H), 9.07 (d, J = 2.5 Hz, 1H), 8.94 (d, J = 4.2 Hz, 1H), 8.66 (d, J = 8.7 Hz, 1H), 8.60 (d, J = 7.9 Hz, 1H), 8.47 (dd, J = 8.7, 2.6 Hz, 1H), 8.16 (td, J = 7.8, 1.8 Hz, 1H), 7.73 (ddd, J = 7.7, 4.7, 1.2 Hz, 1H), 3.62 (m, 6H) ppm. ¹³C NMR (101 MHz, DMSO-d6): δ 173.86, 172.77, 171.68, 163.55, 163.24, 151.09, 150.71, 144.69, 141.66, 138.33, 138.28, 127.08, 126.50, 125.46, 124.69, 59.58, 56.45 ppm. ESI-MS: m/z Calc. for C₁₈H₁₆N₈O₅ 424.12 Da; Obs. [M+H]⁺ 425.33 Da and [M-H]⁻ 423.42 Da.

### Example 3: Inhibition of physiologically relevant target proteins by tetrazines

In this Example, the inhibition of several physiologically relevant target proteins (viz. enzymes and a transporter) by tetrazines were tested *in vitro.* The enzymes tested were cyclooxygenase (COX-1), acetyl cholinesterase (ACES), and monoamine oxidase (MAO-B), and the tested transporter was calcium channel L-type, dihydropyridine. COX-1, ACES, and MAO-B from humans was used, while calcium channel L-type, dihydropyridine from rats was used. Standard literature protocols were used, and relevant controls were included to ensure the validity of the results. The target proteins were mixed with tetrazine **2.1** (reference), **2.4, 2.5, 2.7,** or **2.10** (final concentration of tetrazine 10 µM) in appropriate buffers. The final concentrations for the respective target proteins depended on the standard protocol used, but for each individual target protein this concentration was the same for the different tetrazines tested. After an appropriate incubation time at an appropriate temperature, the enzymatic activity of the enzyme or the ligand binding in case of the transporter was tested in accordance with standard procedures from literature. From this remaining enzymatic activity or ligand binding, the percentage of inhibition was determined.

The results of these experiments are presented in Table 1 below. Therein, it is clear that reference compound **2.1** typically leads to a higher inhibition of the target protein activity as compared to the tetrazines of Formula (1), which show a lower inhibition. This advantageous effect of the compounds of Formula (1) is especially apparent when looking at the inhibition of COX-1, ACES, and calcium channel L-type, dihydropyridine.

**Table 1. Results from the enzyme inhibition studies.**

| **Tetrazine** | **% COX-1 inhibition** | **% MAO-B inhibition** | **% ACES inhibition** | **% Calcium channel L-type dihydropyridine inhibition** |
|---|---|---|---|---|
| **2.1 (reference)** | 59 | 36 | 44 | 44 |
| **2.4** | 53 | 20 | 11 | 10 |
| **2.5** | 18 | 9 | -1 | 1 |
| **2.7** | 37 | 38 | 22 | -17 |
| **2.10** | 14 | 22 | 5 | -3 |

### Example 4: maximum tolerated dose (MTD) of tetrazines in mice

In this Example, groups of Swiss albino mice (an equal number of male and female animals) were subjected to a single dose of tetrazine **2.1** (reference), **2.4, 2.5, 2.7,** or **2.10.** Different groups were used for different doses. For example, for the doses 8, 39, and 78 µmol/kg of reference compound **2.1** three different groups of mice were used. Control groups were also used, wherein mice were only administered vehicle (phosphate-buffered saline (PBS) pH 7.4) without tetrazine ("vehicle control").

Mice were treated in line with ethical guidelines. For example, mice were maintained and monitored for good health at the discretion of a laboratory animal veterinarian, certified rodent diet was provided *ad libitum,* and water was available *ad libitum.* Environmental controls for the animal room were set to maintain a temperature of 22 to 25°C, humidity of 30-70% RH, and a 12-h light/12-h dark cycle.

The dose formulations were prepared as follows. Tetrazine **2.1** (reference), **2.4, 2.5, 2.7,** or **2.10** was dissolved in PBS pH 7.4 to prepare separate stock solutions of each tetrazine. The pH of the above solutions was adjusted with 2M sodium carbonate to 7.17. Further dilutions of the tetrazine stock solutions were done with PBS pH 7.4 to achieve a desired concentration.

Mice were dosed on day 1 by intravenously administering the dose formulation (expressed in µmol of tetrazine per kg body weight of the mouse). Thereafter, the mice were observed up to 72 hours post-dose for any signs of mortality. The number of dead mice after 72 hours ("mortality") was then divided by the original number of mice.

The results of these experiments are summarized in Table 2 below. Therein, it is clear that the maximum tolerated dose of reference compound **2.1** is about 39 µmol/kg. By contrast, for the tetrazines of Formula (1) **(2.4, 2.5, 2.7,** and **2.10)** the maximum tolerated dose is at least 57 µmol/kg. The improvement as compared to reference compound **2.1** is especially evident for compounds **2.5, 2.7,** and **2.10** which have MTD's of at least 236 µmol/kg.

**Table 2. Results from maximum tolerated dose studies in mice.**

| **Tetrazine** | **Tetrazine dose (µmol/kg)** | **No. of mortality vs. total no. of mice** |
|---|---|---|
| **No tetrazine (vehicle control)** | N/A | 0 out of 6 |
| **2.1 (reference)** | 8 | 0 out of 6 |
| | 39 **(MTD)** | 0 out of 6 |
| | 78 | 4 out of 6 |
| **2.4** | 19 | 0 out of 6 |
| | 57 **(MTD)** | 0 out of 6 |
| | 95 | 4 out of 4 |
| **2.5** | 85 | 0 out of 6 |
| | 171 | 0 out of 6 |
| | 512 **(MTD)** | 0 out of 6 |
| **2.7** | 103 | 0 out of 6 |
| | 207 | 0 out of 6 |
| | 621 **(MTD)** | 0 out of 6 |
| **2.10** | 118 | 0 out of 6 |
| | 177 | 0 out of 6 |
| | 236 **(MTD)** | 0 out of 6 |

### Example 5: general in vitro and in vivo properties

Compounds **2.4, 2.5, 2.7,** and **2.10** of Formula (1) were also tested for their:
i. *in vitro* and/or *in vivo* reactivity towards *trans*-cyclooctenes in relation to payload release from said *trans*-cyclooctene;
ii. *in vitro* stability in mouse, rat, and human plasma;
iii. *in vitro* stability in presence of mouse, rat, and human microsomes;
iv. cell toxicity using LS174T colon carcinoma cells;
v. passive membrane permeability at pH 7.4 using a Parallel Artificial Membrane Permeability Assay; and/or
vi. genotoxicity (*i.e.* mutagenecitiy) in *S. typhimurium* strains TA98, TA100, TA1535 and 1537; and in *E. coli* strain WP2 uvrA[pKM101] in presence or absence of S9.

All experiments were carried out using standard protocols known in the art. In all cases, the compounds of Formula (1) showed desired results. In particular, these results were comparable to those obtained for **2.1** when **2.1** was subjected to the same tests i-vi.

## Claims

1. A compound or a salt, hydrate, or solvate thereof; wherein said compound has a structure according to Formula (1): 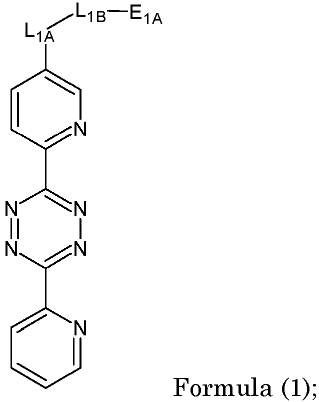 wherein
L_{1A} and L_{1B} are independently selected linkers;
E_{1A} is selected from the group consisting of: 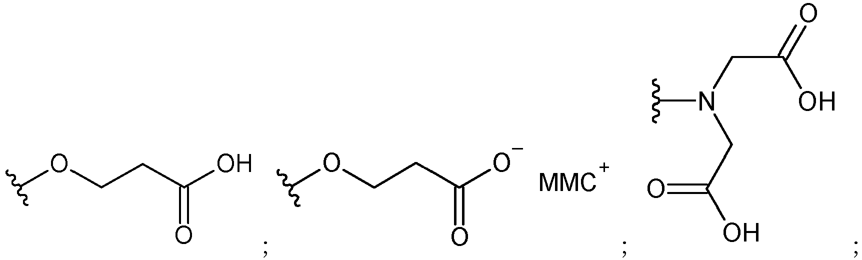 and 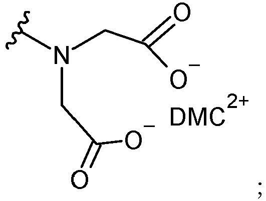 wherein
MMC⁺ is a monovalent metal cation;
DMC²⁺ is a divalent metal cation;
with the proviso that the compound of Formula (1) is not 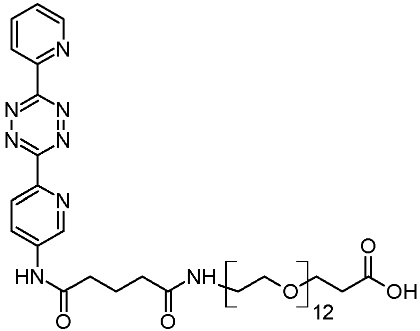 or a salt, solvate, or hydrate thereof;
preferably MMC⁺ is Na⁺;
preferably DMC²⁺ is Ca²⁺.

2. The compound according to claim 1, or the salt, hydrate, or solvate thereof; wherein L_{1A} is a linker containing at most 10 atoms.

3. The compound according to any one of the preceding claims, or the salt, hydrate, or solvate thereof; wherein L_{1A} is selected from the group consisting of -O-, -S-, -SS-, -NR_{L1A}-,
-N=N-, -C(O)-, -C(O)NR_{L1A}-, -OC(O)-, -C(O)O-, -OC(O)NR_{L1A}-, -NR_{L1A}C(O)-, -NR_{L1A}C(O)O-, -NR_{L1A}C(O)NR₄-, -SC(O)-, -C(O)S-, -SC(O)O-, -OC(O)S-, - SC(O)NR_{L1A}-, and -NR_{L1A}C(O)S-; wherein
R_{L1A} is hydrogen or C₁₋₃ alkyl;
preferably L_{1A} is -NR_{L1A}C(O)-;
preferably R_{L1A} is hydrogen.

4. The compound according to any one of the preceding claims, or the salt, hydrate, or solvate thereof; wherein L_{1B} is a linker containing at most 50 atoms;
preferably L_{1B} is a linker containing at most 30 atoms;
more preferably L_{1B} is a linker containing at most 25 atoms;
most preferably L_{1B} is a linker containing at most 10 atoms.

5. The compound according to any one of the preceding claims, or the salt, hydrate, or solvate thereof; wherein L_{1B} is selected from the group consisting of -CH₂-, 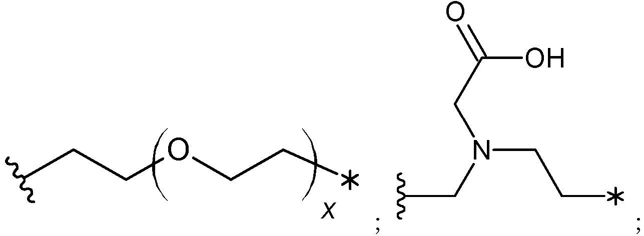 and 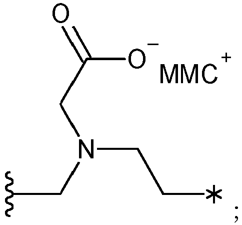 wherein
the wiggly line indicates a bond to L_{1A} and the asterisk a bond to E_{1A};
x is an integer in a range of from 1 to 12;
preferably x is an integer in a range of from 2 to 7;
more preferably x is an integer in a range of from 2 to 4.

6. The compound according to any one of the preceding claims, or the salt, hydrate, or solvate thereof; wherein L_{1B} and E_{1A} together form a moiety selected from the group consisting of: 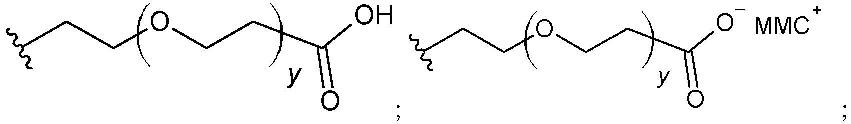 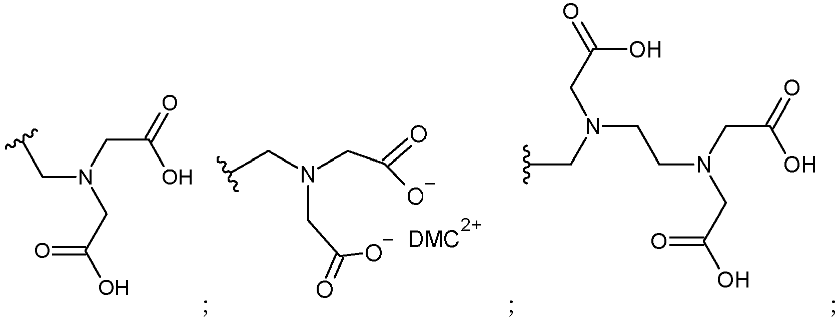 and 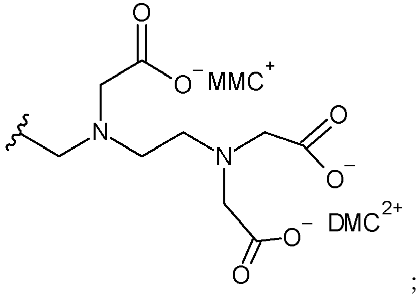 wherein
y is an integer in a range of from 1 to 13;
preferably y is an integer in a range of from 2 to 6;
more preferably y is an integer in a range of from 2 to 4.

7. The compound according to any one of the preceding claims, or the salt, hydrate, or solvate thereof, wherein said compound is selected from the group consisting of:

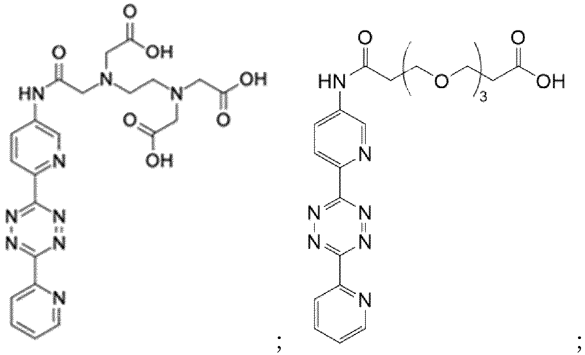

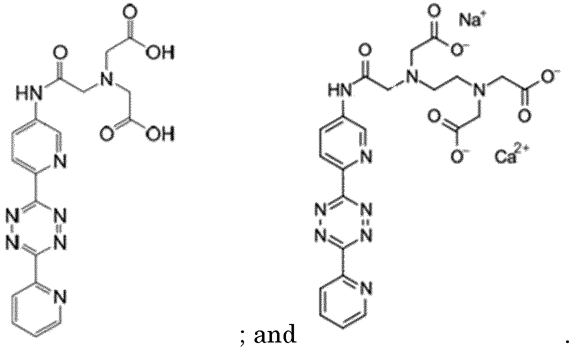

8. The compound according to any one of the preceding claims, or the salt, hydrate, or solvate thereof, wherein said compound is:

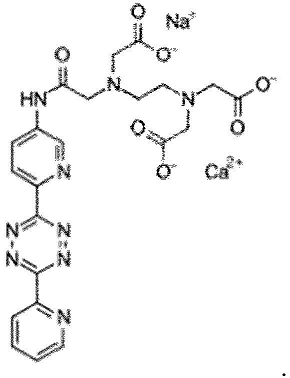

9. The compound according to any one of claims 1 to 7, or the salt, hydrate, or solvate thereof, wherein said compound is:

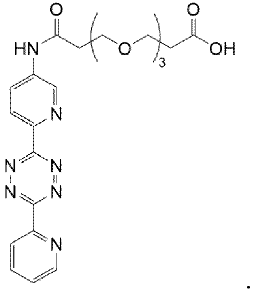

10. The compound according to any one of claims 1 to 7, or the salt, hydrate, or solvate thereof, wherein said compound is:

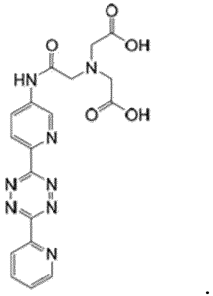

11. A composition comprising a compound according to any one of claims 1 to 10, or the salt, hydrate, or solvate thereof;
preferably the composition is a pharmaceutical composition.

12. A combination of
(A1) a compound according to any one of claims 1 to 10, or the salt, hydrate, or solvate thereof; and/or
(A2) a composition according to claim 11; with
(B) a dienophile or a salt, solvate, or hydrate thereof;
preferably the dienophile comprises a *trans*-cyclooctene moiety.

13. The compound according to any one of claims 1 to 10, or the salt, hydrate, or solvate thereof; the composition according to claim 11; or the combination according to claim 12; for use as a medicament.

14. The compound according to any one of claims 1 to 10, or the salt, hydrate, or solvate thereof; the composition according to claim 11; or the combination according to claim 12; for use in the treatment of a disease in a subject,
preferably the subject is a human;
preferably the disease is cancer.

15. A method for preparing a compound according to claim 7, wherein said method comprises the steps of:
(a) reacting SM1a or SM1b with a reagent selected from the group consisting of SM2, SM3, and SM4;
(b) if SM1a is used in step (a), subjecting the reaction product of step (a) to oxidation;
(c) optionally, subjecting the reaction product of step (a) or step (b) to salt formation;
wherein SM1a, SM1b, SM2, SM3, and SM4 are: 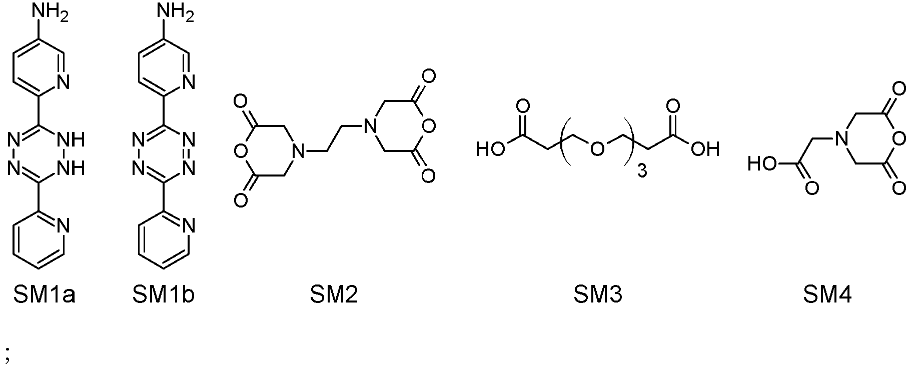
preferably in step (a) SM1a is used;
preferably in step (b) the reaction product of step (a) is contacted with sodium nitrite.

16. A non-therapeutic method for reacting:
(ia) the compound according to any one of claims 1 to 10, or the salt, hydrate, or solvate thereof; and/or
(iia) the composition according to claim 11;
with a dienophile or a salt, solvate, or hydrate thereof,
wherein said method comprises the step of contacting (ia), and/or (iia) with said dienophile or salt, solvate, or hydrate thereof,
preferably said non-therapeutic method is an *in vitro* method; and
preferably said dienophile comprises a *trans*-cyclooctene moiety.

17. A non-therapeutic use of:
(a) the compound according to any one of claims 1 to 10, or the salt, hydrate, or solvate thereof;
(b) the composition according to claim 11; and/or
(c) the combination according to claim 12;
in a click reaction.

## Patentansprüche

1. Verbindung oder ein Salz, Hydrat oder Solvat davon; wobei die Verbindung eine Struktur gemäß Formel (1) hat: 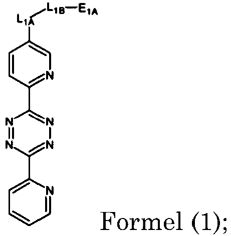 wobei
L_{1A} und L_{1B} unabhängig ausgewählte Linker sind;
E_{1A} ausgewählt ist aus der Gruppe, bestehend aus: 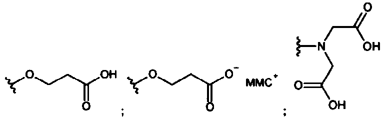 und 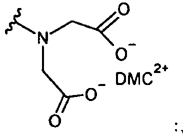 wobei
MMC⁺ ein monovalentes Metallkation ist;
DMC²⁺ ein divalentes Metallkation ist;
unter der Bedingung, dass die Verbindung von Formel (1) nicht 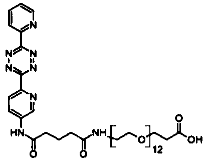 oder ein Salz, Solvat oder Hydrat davon ist;
MMC⁺ vorzugsweise Na⁺ ist;
DMC²⁺ vorzugsweise Ca²⁺ ist.

2. Verbindung nach Anspruch 1 oder das Salz, Hydrat oder Solvat davon; wobei L_{1A} ein Linker ist, der höchstens 10 Atome enthält.

3. Verbindung nach einem der vorhergehenden Ansprüche oder das Salz, Hydrat oder Solvat davon; wobei L_{1A} ausgewählt ist aus der Gruppe, bestehend aus -O-, -S-, -SS-, -NR _{L1A} -,
-N=N-, -C(O)-, -C(O)NR_{L1A}-, -OC(O)-, -C(O)O-, -OC(O)NR_{L1A}-, - NR_{L1A}C(O)-, -NR_{L1A}C(O)O-, -NR_{L1A}C(O)NR₄-, -SC(O)-, -C(O)S-, -SC(O)O-, - OC(O)S-, -SC(O)NR_{L1A} - und -NR_{L1A} C(O)S-; wobei
R_{L1A} Wasserstoff oder C₁₋₃-Alkyl ist;
L_{1A} vorzugsweise -NR_{L1A} C(O)- ist;
R L_{1A} vorzugsweise Wasserstoff ist.

4. Verbindung nach einem der vorhergehenden Ansprüche oder das Salz, Hydrat oder Solvat davon; wobei L_{1B} ein Linker ist, enthaltend höchstens 50 Atome;
L_{1B} vorzugsweise ein Linker ist, enthaltend höchstens 30 Atome;
L_{1B} bevorzugter ein Linker ist, enthaltend höchstens 25 Atome t;
L_{1B} am bevorzugtesten ein Linker ist, enthaltend höchstens 10 Atome.

5. Verbindung nach einem der vorhergehenden Ansprüche oder das Salz, Hydrat oder Solvat davon; wobei L_{1B} ausgewählt ist aus der Gruppe, bestehend aus -CH₂ -, 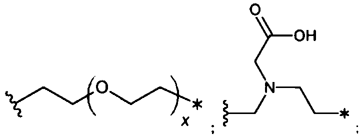 und
wobei 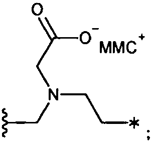
die Wellenlinie eine Bindung an L_{1A} angibt und das Sternchen eine Bindung an E_{1A};
x eine ganze Zahl in einem Bereich von von 1 bis 12 ist;
x vorzugsweise eine ganze Zahl in einem Bereich von von 2 bis 7 ist;
x bevorzugter eine ganze Zahl in einem Bereich von von 2 bis 4 ist.

6. Verbindung nach einem der vorhergehenden Ansprüche oder das Salz, Hydrat oder Solvat davon, wobei L_{1B} und E_{1A} zusammen einen Rest bilden ausgewählte aus der Gruppe, bestehend aus: 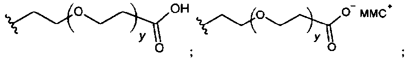 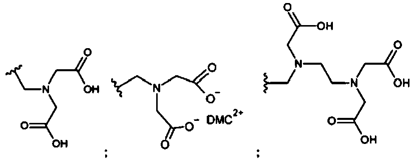 und 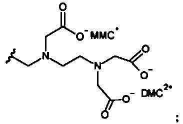 wobei
y eine ganze Zahl in einem Bereich von von 1 bis 13 ist;
y vorzugsweise eine ganze Zahl in einem Bereich von von 2 bis 6 ist;
y bevorzugter eine ganze Zahl in einem Bereich von von 2 bis 4 ist.

7. Verbindung nach einem der vorhergehenden Ansprüche oder das Salz, Hydrat oder Solvat davon; wobei die Verbindung ausgewählt ist aus der Gruppe, bestehend aus

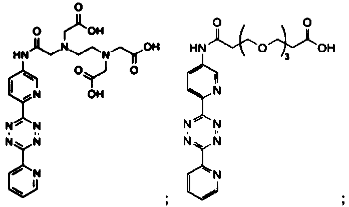

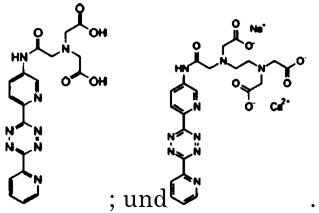

8. Verbindung nach einem der vorhergehenden Ansprüche oder das Salz, Hydrat oder Solvat davon; wobei die Verbindung ist:

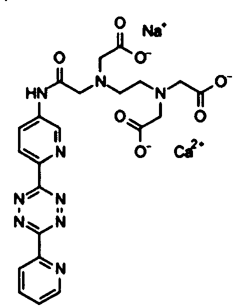

9. Verbindung nach einem der Ansprüche 1 bis 7 oder das Salz, Hydrat oder Solvat davon; wobei die Verbindung ist:

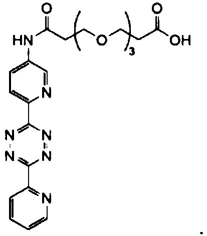

10. Verbindung nach einem der Ansprüche 1 bis 7 oder das Salz, Hydrat oder Solvat davon; wobei die Verbindung ist:

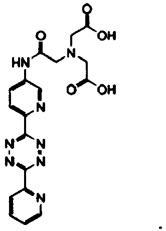

11. Zusammensetzung, umfassend eine Verbindung nach einem der Ansprüche 1 bis 10 oder das Salz, Hydrat oder Solvat davon;
wobei die Zusammensetzung vorzugsweise eine pharmazeutische Zusammensetzung ist.

12. Kombination aus
(A1) einer Verbindung nach einem der Ansprüche 1 bis 10 oder dem Salz, Hydrat oder Solvat davon; und/oder
A2) einer Zusammensetzung nach Anspruch 11; mit
(B) einem Dienophil oder einem Salz, Solvat oder Hydrat davon;
wobei das Dienophil vorzugsweise einen *Trans*-Cycloocten-Rest umfasst.

13. Verbindung nach einem der Ansprüche 1 bis 10 oder das Salz, Hydrat oder Solvat davon; die Zusammensetzung nach Anspruch 11; oder die Kombination nach Anspruch 12; zur Verwendung als Arzneimittel.

14. Verbindung nach einem der Ansprüche 1 bis 10 oder das Salz, Hydrat oder Solvat davon; die Zusammensetzung nach Anspruch 11; oder die Kombination nach Anspruch 12; zur Verwendung bei der Behandlung einer Krankheit bei einem Subjekt,
vorzugsweise ist das Subjekt ein Mensch;
vorzugsweise ist die Krankheit Krebs.

15. Verfahren zum Herstellen einer Verbindung nach Anspruch 7, wobei das Verfahren die Schritte umfasst von:
a) Reagieren von SM1a oder SM1b mit einem Reagens, ausgewählt aus der Gruppe bestehend aus SM2, SM3 und SM4;
(b) wenn SM1a in Schritt a) verwendet wird, Unterzeiehen des Reaktionsprodukts von Schritt a) einer Oxidation;
(c) optional Unterziehen des Reaktionsprodukts von Schritt a) oder Schritt b) einer Salzbildung;
wobei SM1a, SM1b, SM2, SM3 und SM4 sind: 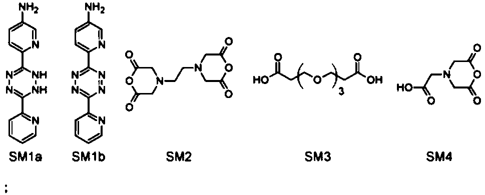
vorzugsweise wird in Schritt a) SM1a verwendet;
vorzugsweise wird in Schritt (b) das Reaktionsprodukt von Schritt (a) mit Natriumnitrit in Kontakt gebracht.

16. Nichttherapeutisches Verfahren zum Reagieren:
(ia) der Verbindung nach einem der Ansprüche 1 bis 10 oder des Salzes, Hydrats oder Solvats davon; und/oder
iia) der Zusammensetzung nach Anspruch 11;
mit einem Dienophil oder einem Salz, Solvat oder Hydrat davon,
wobei das Verfahren den Schritt des in Kontaktbringens von (ia), und/oder (iia) mit dem Dienophil oder dem Salz, Solvat oder Hydrat davon umfasst,
vorzugsweise das nichttherapeutische Verfahren ein *In-vitro-*Verfahren ist; und das Dienophil vorzugsweise einen *Trans*-Cycloocten-Rest umfasst.

17. Nichttherapeutische Verwendung von:
(a) der Verbindung nach einem der Ansprüche 1 bis 10 oder des Salzes, Hydrats oder Solvats davon;
b) der Zusammensetzung nach Anspruch 11; und/oder
c) der Kombination nach Anspruch 12;
in einer Click-Reaktion.

## Revendications

1. Composé, ou sel, hydrate ou solvate de celui-ci ; dans lequel ledit composé a une structure selon la formule (1) : 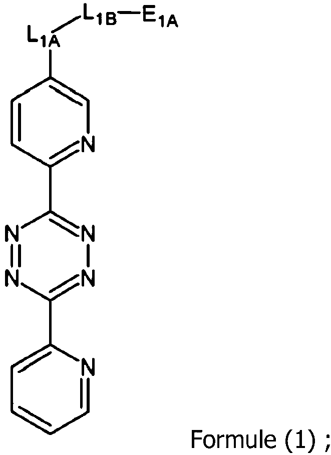 dans lequel
L_{1A} et L_{1B} sont des lieurs indépendamment choisis ;
E_{1A} est choisi dans le groupe constitué par : 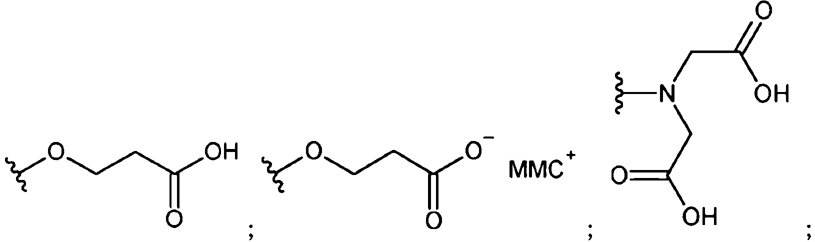
et 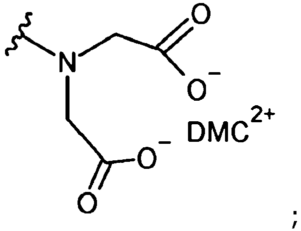 dans lequel
MMC⁺ est un cation métallique monovalent ;
DMC²⁺ est un cation métallique divalent ;
à condition que le composé de formule (1) ne soit pas 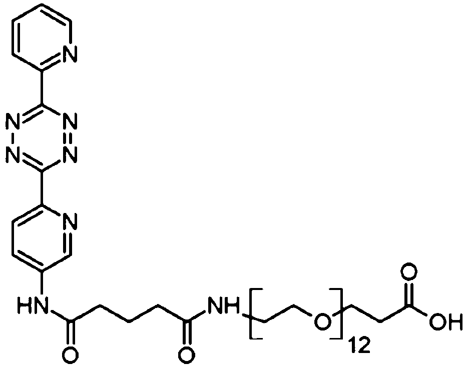 ou un sel, un hydrate ou un solvate de celui-ci ;
de préférence MMC⁺ est Na⁺ ;
de préférence DMC²⁺ est Ca²⁺.

2. Composé selon la revendication 1, ou sel, hydrate ou solvate de celui-ci ; dans lequel L_{1A} est un lieur contenant au plus 10 atomes.

3. Composé selon l'une quelconque des revendications précédentes, ou sel, hydrate ou solvate de celui-ci ; dans lequel L_{1A} est choisi dans le groupe constitué par -O-, -S-, -SS-, -NR_{L1A}-, -N=N-, -C(O)-, -C(O)NR_{L1A}-, -OC(O)-, - C(O)O-, -OC(O)NR_{L1A}-, -NR_{L1A}C(O)-, -NR_{L1A}C(O)O-, -NR_{L1A}C(O)NR₄-, -SC(O)-, - C(O)S-, -SC(O)O-, -OC(O)S-, -SC(O)NR_{L1A}- et -NR_{L1A}C(O)S- ; dans lequel
R_{L1A} est un atome d'hydrogène ou un groupe alkyle en C₁₋₃ ;
de préférence L_{1A} est -NR_{L1A}C(O)- ;
de préférence R_{L1A} est un atome d'hydrogène.

4. Composé selon l'une quelconque des revendications précédentes, ou sel, hydrate ou solvate de celui-ci ; dans lequel L_{1B} est un lieur contenant au plus 50 atomes ;
de préférence, L_{1B} est un lieur contenant au plus 30 atomes ;
plus préférablement, L_{1B} est un lieur contenant au plus 25 atomes ;
de manière préférée entre toutes, L_{1B} est un lieur contenant au plus 10 atomes.

5. Composé selon l'une quelconque des revendications précédentes, ou sel, hydrate ou solvate de celui-ci ; dans lequel L_{1B} est choisi dans le groupe constitué par -CH₂-, 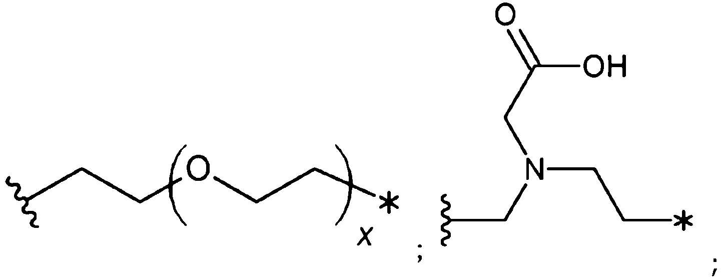 et 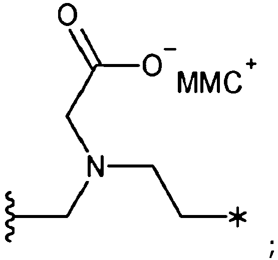 dans lequel
la ligne ondulée indique une liaison avec L_{1A} et l'astérisque une liaison avec E_{1A} ;
x est un nombre entier dans la plage de 1 à 12 ;
de préférence, x est un nombre entier dans la plage de 2 à 7 ;
plus préférablement, x est un nombre entier dans la plage de 2 à 4.

6. Composé selon l'une quelconque des revendications précédentes, ou sel, hydrate ou solvate de celui-ci ; dans lequel L_{1B} et E_{1A} forment ensemble un fragment choisi dans le groupe constitué par : 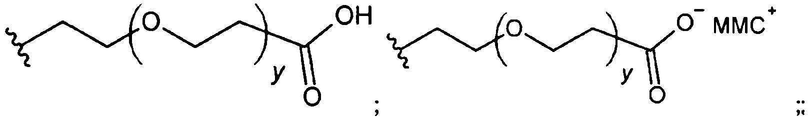 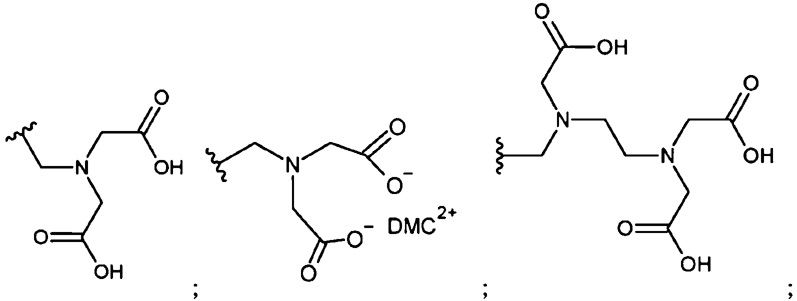 et 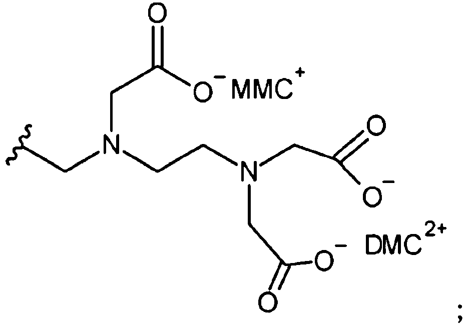 dans lequel
y est un nombre entier dans la plage de 1 à 13 ;
de préférence, y est un nombre entier dans la plage de 2 à 6 ;
plus préférablement, y est un nombre entier dans la plage de 2 à 4.

7. Composé selon l'une quelconque des revendications précédentes, ou sel, hydrate ou solvate de celui-ci, dans lequel ledit composé est choisi dans le groupe constitué par :

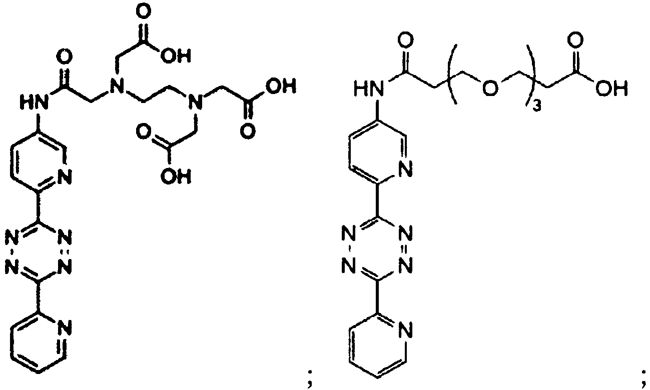

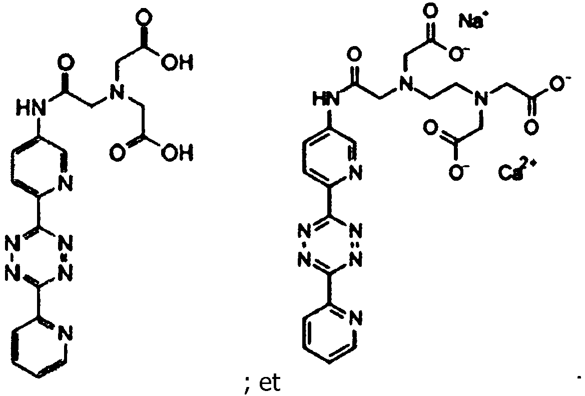

8. Composé selon l'une quelconque des revendications précédentes, ou sel, hydrate ou solvate de celui-ci, dans lequel ledit composé est :

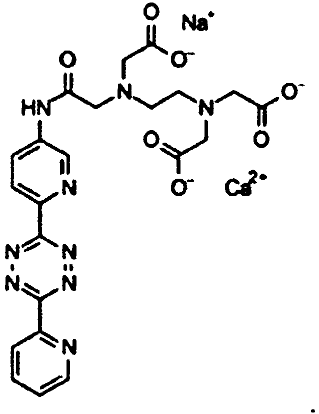

9. Composé selon l'une quelconque des revendications 1 à 7, ou sel, hydrate ou solvate de celui-ci, dans lequel ledit composé est :

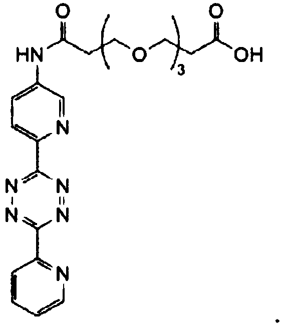

10. Composé selon l'une quelconque des revendications 1 à 7, ou sel, hydrate ou solvate de celui-ci, dans lequel ledit composé est :

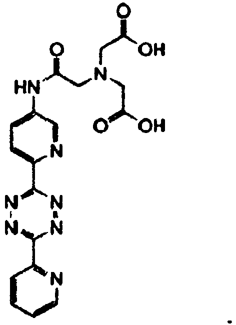

11. Composition comprenant un composé selon l'une quelconque des revendications 1 à 10, ou un sel, un hydrate ou un solvate de celui-ci ;
de préférence, la composition est une composition pharmaceutique.

12. Association de
(A1) un composé selon l'une quelconque des revendications 1 à 10, ou un sel, un hydrate ou un solvate de celui-ci ;et/ou
(A2) une composition selon la revendication 11 ; avec
(B) un diénophile ou un sel, un solvate ou un hydrate de celui-ci ;
de préférence, le diénophile comprend un fragment *trans-*cyclooctène.

13. Composé selon l'une quelconque des revendications 1 à 10, ou sel, hydrate ou solvate de celui-ci ; composition selon la revendication 11 ; ou association selon la revendication 12 ; pour une utilisation comme médicament.

14. Composé selon l'une quelconque des revendications 1 à 10, ou sel, hydrate ou solvate de celui-ci ; composition selon la revendication 11 ; ou association selon la revendication 12 ; pour une utilisation dans le traitement d'une maladie chez un sujet
de préférence le sujet est un être humain ;
de préférence, la maladie est un cancer.

15. Procédé de préparation d'un composé selon la revendication 7, dans lequel le procédé comprend les étapes consistant à :
a) faire réagir SM1a ou SM1b avec un réactif choisi dans le groupe constitué par SM2, SM3 et SM4 ;
b) si SM1a est utilisé à l'étape a), soumettre le produit de la réaction de l'étape a) à une oxydation ;
c) éventuellement, soumettre le produit de la réaction de l'étape a) ou de l'étape b) à la formation d'un sel ;
dans lequel SM1a, SM1b, SM2, SM3 et SM4 sont : 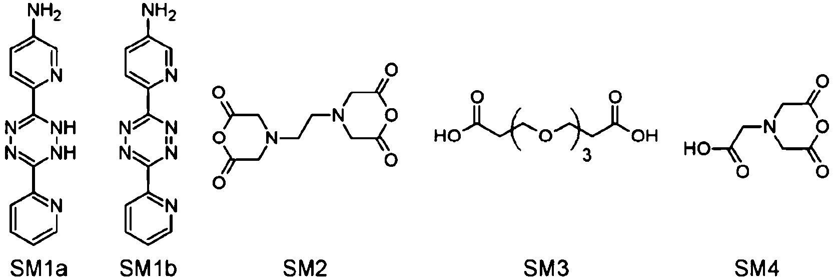
de préférence, SM1a est utilisé à l'étape (a) ;
de préférence, le produit de la réaction de l'étape (a) est mis en contact avec du nitrite de sodium à l'étape (b).

16. Procédé non thérapeutique de réaction :
(ia) du composé selon l'une quelconque des revendications 1 à 10, ou d'un sel, d'un hydrate ou d'un solvate de celui-ci ; et/ou
(iia) de la composition selon la revendication 11 ;
avec un diénophile ou un sel, un solvate ou un hydrate de celui-ci,
dans lequel ledit procédé comprend l'étape consistant à mettre en contact (ia) et/ou (iia) avec ledit diénophile ou un sel, un solvate ou un hydrate de celui-ci ;
de préférence, ledit procédé non thérapeutique est un procédé *in vitro* ; et
de préférence, ledit diénophile comprend un fragment *trans-cyclooctène.*

17. Utilisation non thérapeutique:
(a) du composé selon l'une quelconque des revendications 1 à 10, ou d'un sel, d'un hydrate ou d'un solvate de celui-ci ;
(b) de la composition selon la revendication 11 ; et/ou
(c) de l'association selon la revendication 12 ;
dans une réaction click.
